(19) Europäisches Patentamt
European Patent Office
Office européen des brevets

(11) Publication number: 0 390 674
A1

(12) # EUROPEAN PATENT APPLICATION

(21) Application number: 90400835.6

(22) Date of filing: 28.03.90

(51) Int. Cl.5: C07H 15/26, A61K 31/70

(30) Priority: 29.03.89 EP 89400881

(43) Date of publication of application:
03.10.90 Bulletin 90/40

(84) Designated Contracting States:
AT BE CH DE DK ES FR GB GR IT LI LU NL SE

(71) Applicant: MERRELL DOW PHARMACEUTICALS INC.
2110 East Galbraith Road
Cincinnati Ohio 45215-6300(US)

(72) Inventor: Ducep, Jean Bernard
29, rue des Alpes, Sundhoffen
F-68600 Neuf-Brisach(FR)
Inventor: Danzin, Charles
18, rue Geiler
F-67000 Strasbourg(FR)

(74) Representative: Gillard, Marie-Louise et al
Cabinet Beau de Loménie 55, Rue d'Amsterdam
F-75008 Paris(FR)

(54) **Novel alpha-glucosidase inhibitors.**

(57) This invention relates to novel N-derivatives of 1,4-dideoxy-1,4-imino-L-arabinitol, to the processes for their preparation and to their end-use applications, particularly as to their use in the treatment of diabetes.

EP 0 390 674 A1

Xerox Copy Centre

## NOVEL α-GLUCOSIDASE INHIBITORS

This invention relates to novel 1,4-di-deoxy-1,4-imino-L-arabinitol derivatives, to the processes for their preparation and to their end-use applications, particularly as to their use in the treatment of diabetes.

More specifically this invention relates to novel N-glycosyl derivatives of 1,4-dideoxy-1,4-imino-L-arabinitol, to the chemical processes for their preparation, to their α-glucosidase inhibiting properties, and to their end-use application in the treatment of diabetes, obesity and those diseases associated with retroviruses, particularly the HIV virus reported to be the causative of the acquired immune deficiency syndrome (AIDS).

Still more specifically this invention relates to the novel 1,4-dideoxy-1,4-imino-L-arabinitol derivatives of the formula

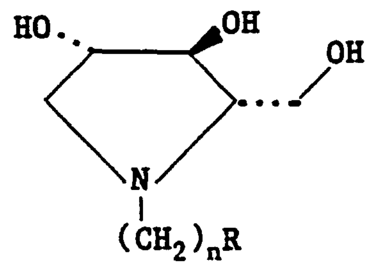

I

and the pharmaceutically acceptable acid addition salts thereof wherein n is 1 or 2 and R is a glycosyl moiety. The glycosyl moiety represented by "R" in Formula I are radicals which contain from 1 to 3 hexose or pentose units which optionally bear an ether or an acyl radical at the anomeric carbon atom.

Acid addition salts are those salts forms with such inorganic acids as, for example, hydrochloric, hydrobromic, sulfuric, phosphoric and like acids; with organic carboxylic acids such as, for example, acetic, propionic, glycolic, lactic, pyruvic, malonic, succinic, fumaric, malic, tartaric, citric, ascorbic, maleic, hydroxymaleic and dihydroxymaleic, benzoic, 2-acetoxybenzoic, madelic and like acids; and with organic sulfonic acids such as methanesulfonic acid and p-toluenesulfonic acid.

In general, the mono-, di- or trisaccharide moiety (i.e., the glycosyl moiety defined by "R") may be attached directly -or thru a $(CH_2)_n$ alkylene bridge to the nitrogen atom of the 1,4-dideoxy-1,4-imino-L-arabinitol moiety thru either an exocyclic or ring carbon atom of the pentose or hexose ring thereby forming a variety of position isomers for each individual glycosyl moiety. Also, similar or dissimilar pentose or hexose moieties may be linked to each other thru a glycosidic oxygen bridge wherein the bridging oxygen atom is attached to an exocylic and/or endocyclic carbon atom of the pentose or hexose moiety of which the glycosyl radical is comprised; again the position isomers all being contemplated as being within the scope of this invention.

Exemplary of glycosyl radicals comtemplated by the "R" designation in Formula I are such monosaccharides as glucosyl, galactosyl, fucosyl, fructosyl, mannosyl, ribosyl, arabinosyl, xylosyl, allosyl, altrosyl, gulosyl, idosyl, talosyl and lyxosyl, such disaccharides as isomaltosyl, trehalosyl, α and β cellobiosyl, maltosyl, and such trisaccharides as maltotriosyl and cellotriosyl. Preferred glycosyl radicals are 6- or 4-glucosyl, 1- or 6-fructosyl, 6- or 4-maltosyl and 6- or 4-isomaltosyl. Ether derivatives are those derivatives wherein the hydroxyl group attached to the anomeric carbon atom is etherified and include the $C_{1-6}$ alkyl derivatives, preferably methyl and aromatic derivatives such as phenyl and benzyl. Acyl derivatives, such as those formed at the anomeric carbon atom by reaction of the free hydroxy radical with alkanoic acids or benzoic acid, are also contemplated even though such acylated moieties may easily be removed from the glycosyl radical. Preferred acyl radicals are those formed with acetic or benzoic acids.

In general, the compounds of Formula I may be prepared by procedures analogously known in the art, said reactions being depicted by the following generic Reaction Schemes A and B.

**Reaction Scheme A:**

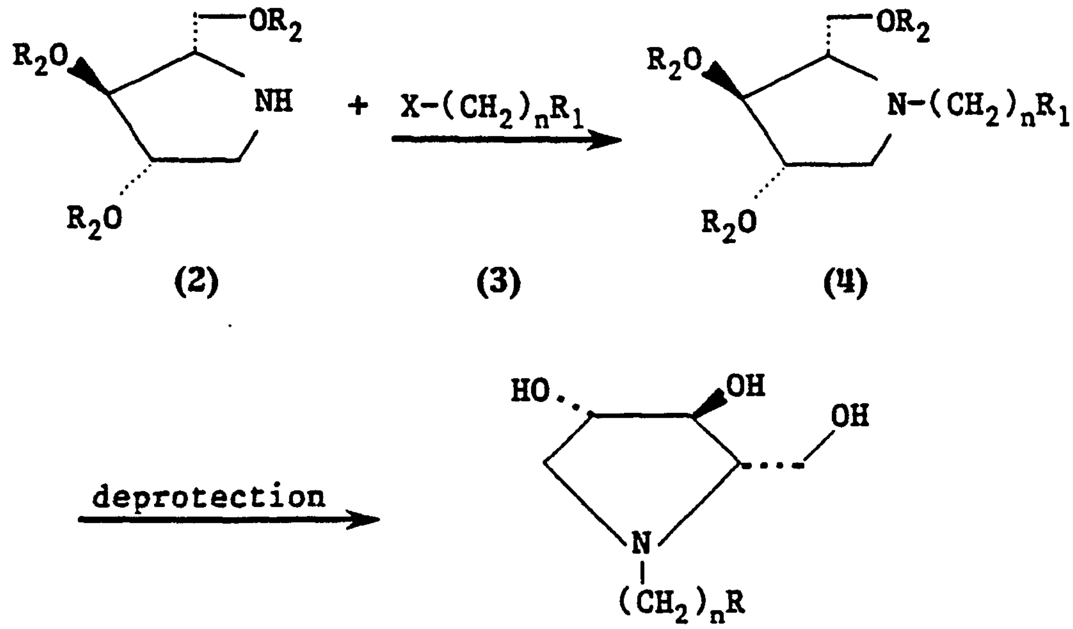


(2) (3) (4)

**Reaction Scheme B:**

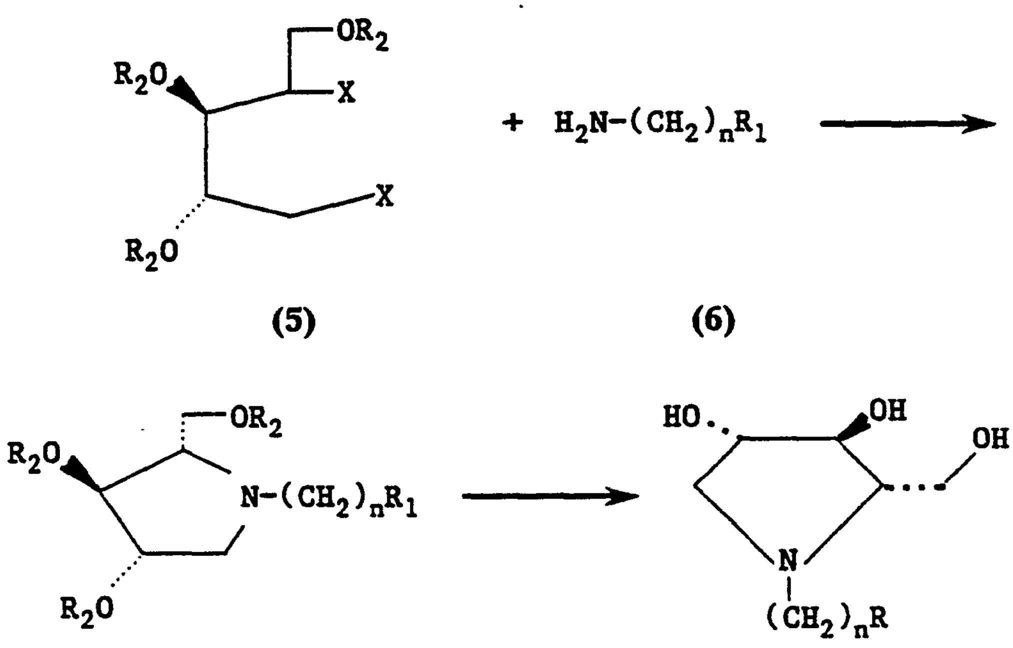


(5) (6)

(4) I

wherein in each of Schemes A and B, compounds of Formula I are as previously defined.

$R_1$ is a glycosyl moiety which optionally has its free hydroxy radicals bearing a reaction-protecting group, said glycosyl moiety containing one to three hexose or pentose units, the terminal anomeric carbon atoms of which may optionally bear an OH, $C_{1-6}$ alkoxy, $-O-\underset{\overset{\parallel}{O}}{C}-C_{1-8}$

alkyl or benzyl moiety,
X is chloro, bromo, iodo or an activated oxygen radical,
n is one or two,
$R_2$ is H or a reaction protecting group, with the proviso that when X is an activated oxygen moiety, $R_2$ is other than H and the free hydroxy radicals of the glycosyl moieties must bear a reaction-protection group. Compounds (5) are modified 1,4-D-xylitol derivatives.

In order to more conveniently teach the preparation of the compounds of Formula I, the generic Reaction Schemes A and B may be illustrated by the following Reaction Schemes C and D.

**Reaction Scheme C:**

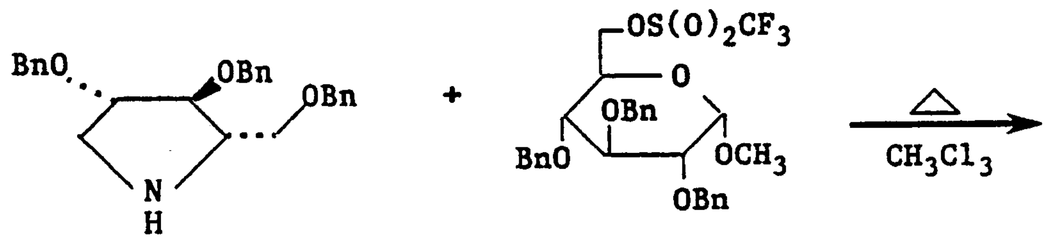


(7) (8)

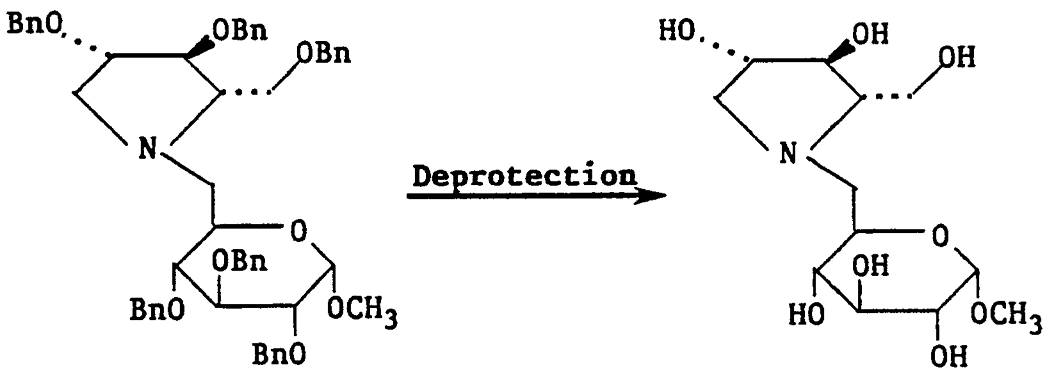


(9) (10)

**Reaction Scheme D:**

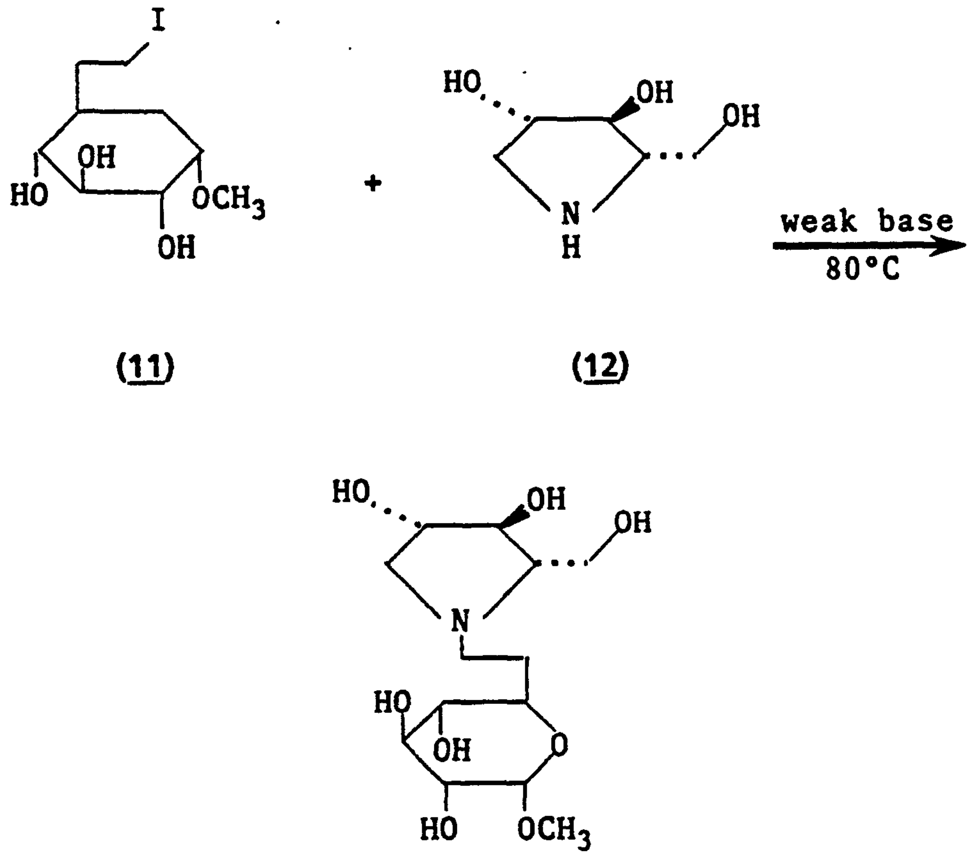


(11) (12)

(13)

wherein, in Scheme C, the triflate (i.e., $-OS(O)_2CF_3$) is representative of an activated oxygen moiety, and in Scheme D, the iodo moiety is representative of the halides of X, and Bn is benzyl which is representative of the hydroxy protecting groups of P'g of Schemes A and B.

In effecting the reactions of Schemes A, C and D, it is preferred to condense an appropriately hydroxy protected 1,4-dideoxy-1,4-imino-L-arabinitol [(2) or (7) or the unprotected (12)] with an appropriately hydroxy protected glycosyl triflate [(3) and (8)] or halide (11), preferably the iodide. In those instances wherein the 1,4-dideoxy-1,4-imino-L-arabinitol is coupled with a triflate the reaction is effected by refluxing an admixture of equimolar quantities of the reactants in an alcohol- and water-free solvent, preferably a chlorinated solvent such as chloroform, under an inert atmosphere, preferably under nitrogen or argon, for about 1 to 3 days until the reaction is completed. Following standard procedures for the isolation and purification of the reaction products, the protecting groups are removed to obtain the desired product. Debenzylation is readily effected with standard techniques such as catalytic hydrogenation in an appropriate solvent, e.g., ethanol, using a catalyst such as palladium or carbon, or by transfer hydrogenation using cyclohexene and methanol. In those instances wherein esters were utilized (partially or completely) as the hydroxy protecting groups, it is preferred to first remove the ester group by treatment with catalytic amounts of a base, e.g., an alkali alkoxide (e.g. sodium methoxide in ethanol) or with ammonia in methanol to hydrolyze the esters and then deprotect the benzyl ethers using the foregoing hydrogenation procedures.

In those instances wherein a glycosyl halide (chloro, bromo or iodo) is coupled with the 1,4-dideoxy-1,4-imino-L-arabinitol the reaction is effected by heating the appropriately hydroxy protected reactants in dry dimethyl formamide (DMF) or other equivalently functioning solvent, at about 60°-90°C for about 12 to 36 hours, said heating taking place using excess amounts of a weak base ($K_1CO_3$) or a molecular sieve, preferably using excess molar amounts of the halide (up to three times) relative to the amine. In this condensation it is not necessary to utilize a hydroxy protecting group but, if utilized, although benzyl is preferred, other well known protecting groups may also be utilized and are removed as herein described.

Reaction Scheme B is effected by refluxing the reactants together in acetonitrile, using an escess of the amine (6), or in the presence of a tertiary amine, according to the teachings of G.W.J. Fleet and Jong Chan Son (Tetrahedron, Vol. 44, No. 9, pp. 2637 to 2647, 1988).

Appropriately hydroxy protected glycosyl halides (6) and triflates (3) are those glycosyl radicals (mono-, di-, or trisaccarides of Formula I) where in the hydroxy groups have been protected with an ester or ether moiety. Preferred esters are the acetate or benzoate esters although other alkanoyl esters, particularly those containing up to six carbon atoms, may be used. The preferred ether is the benzyl ether. Such protected compounds may be prepared by standard procedures very well known and understood in the art.

The glycosyl triflates (of which compound 3 is representative) are prepared by standard procedures such as by reaction of an hydroxy protected glycosyl with trifluoro-methylsulfonate anhydride in a chlorinated solvent for about 1-3 hours at about -78°C to -10°C.

The glycoside halides (of which compound 6 is representative) may be prepared by standard techniques starting with an appropriately hydroxy protected glycoside bearing one free hydroxy group. In these instances the alcohol is converted to its aldehyde by a Swern oxidation (treatment with oxalyl chloride in dimethylsulfoxide and triethylamine) followed by an *in situ* conversion of the aldehyde to an olefin by a Wittig reaction (going through a "ylide" prepared from methyltriphenylphosphonium bromide using one equivalent each of n-butyllithium, potassium t-butoxide and t-butanol in tetrahydrofuran at room temperature for about 4 to 8 hours). The olefin is converted to its corresponding alcohol by hydroboration (treatment with boron dimethylsulfide, under nitrogen, followed by oxidation with hydrogen peroxide and sodium hydroxide). The alcohol is mesylated (treatment with mesyl chloride in $CH_2Cl_2$ in excess $NEt_3$ at -15°6 to 0°C) and the mesylate converted to its halide (by treatment in ether at 0°C with magnesium halide), preferably using the iodide.

The following examples illustrate the processes and techniques suitable for the preparation of the compounds of this invention.

## Example 1

### Preparation of

### METHYL 2,3,4-TRI-O-BENZYL-6-O-TRIFLUOROMETHYLSULFONYL-α-D-GLUCOPYRANOSIDE

To a solution of dry pyridine (0.46 mL) in methylene chloride (17.5 mL) cooled to -15°C was added trifluoromethanesulfonic anhydride (0.87 mL). The mixture was stirred during 15 min at -10°C, then methyl 2,3,4-tri-O-benzyl-α-D-glucopyranoside (1.2 g, 2.58 mmol) in methylene chloride (5 mL)was added [P. Kovac, V. Sklenar and 6. Glaudemans, Carbohydr. Res. 175, 201 (1988)]. The mixture was stirred during 1.5 h at -10°C. The reaction mixture was washed with water. The organic layer was dried over sodium sulfate, filtered and concentrated under reduced pressure to afford an oil. Flash chromatography on silica gel and elution with a 7:3 mixture of hexane and ethyl acetate afforded the expected compound methyl 2,3,4-tri-O-benzyl-6-O-trifluoromethylsulfonyl-α-D-glucopyranoside which was crystallized from hexane (1.43 g, 93%); m.p. 44-45°C.

## Example 2

### Preparation of

### 2,3,5-TRI-O-BENZYL-1,4-DIDEOXY-1,4-[(2,3,4-TRI-O-BENZYL-6-DEOXY-1-O-METHYL-6-α-D-GLUCOPYRANOSYL)IMINO]-L-ARABINITOL

A solution of methyl 2,3,4-tri-O-benzyl-6-O-trifluoromethylsulfonyl-α-D-glucopyranoside (0.903 g, 1.52 mmol) and 2,3,5-tri-O-benzyl-1,4-dideoxy-1,4-imino-L-arabinitol [G.W.J. Fleet, S.J. Nicholas, P.W. Smith, S.V. Evans, L.E. Fellows and R.J. Nash, Tetrahedron Letters, 26, 3127, 1985] (0.611 g, 1.52 mmol) in ethanol-free chloroform (55 mL) was refluxed under nitrogen during 48 h. The mixture was diluted in methylene chloride and washed successively with a saturated aqueous solution of sodium bicarbonate and saturated brine. The organic layer was dried over sodium sulfate, filtered and concentrated under reduced pressure to afford an oil. Flash chromatography on silica gel and elution with a 6:4 mixture of hexane and ethyl acetate afforded the expected compound 2,3,5-tri-O-benzyl-1,4-dideoxy-1,4-[(2,3,4-tri-O-benzyl-6-deoxy-1-O-methyl-6-α-D-glucopyranosyl)imino]-L-arabinitol, as a white foam (0.9 g, 70%).

## Example 3

Preparation of

1,4-DIDEOXY-1,4-[(6-DEOXY-1-O-METHYL-6-α-D-GLUCOPYRANOSYL)-IMINO]-L-ARABINITOL

2,3,5-tri-O-benzyl-1,4-dideoxy-1,4-[(2,3,4-tri-O-benzyl-6-deoxy-1-O-methyl-6-α-D-glucopyranosyl)imino]-L-arabinitol (0.773 g, 0.91 mmol) was dissolved in a 1:1 mixture of methanol and acetic acid (40 mL) and Pd 10% on charcoal (70 mg) was added. The mixture was stirred under hydrogen at atmospheric pressure during 4 days. The catalyst was filtered off and the solvents were evaporated under reduced pressure. The residue was dissolved in water and passed through a column of Amberlyst A26 $OH^{\ominus}$ form. Lyophilization afforded 1,4-dideoxy-1,4-[(6-deoxy-1-O-methyl-6-α-D-glucopyranosyl)imino]-L-arabinitol as an amorphous solid (0.118 g, 40%).

## Example 4

Preparation of

METHYL 2,3,4-TRI-O-BENZYL-6,7-DIDEOXY-α-D-GLUCOHEPT-6-ENOPYRANOSIDE

To a solution of oxalyl chloride (1.05 mL, 17.22 mmol) in dry tetrahydrofuran (40 mL) cooled to -78° C, dry dimethyl sulfoxyde (1.3 mL, 18.04 mmol) was added dropwise and then stirred during 35 min at -35° C. The reaction mixture was cooled again to -78° C and methyl 2,3,4-tri-O-benzyl-α-D-glucopyranoside (6 g, 16.4 mmol) dissolved in tetrahydrofuran (20 mL) was added and the mixture was stirred during 15 min at -35° C, then triethylamine (11.5 mL, 82.65 mmol) was added and the mixture was stirred during 1 h at -35° C. This aldehyde was used without purification and isolation in a wittig reaction described as follows. To dried triphenylmethylphosphonium bromide (11.7 g, 32.8 mmol) suspended in tetrahydrofuran (700 mL) was added dropwise at -78° C a 1.42 M solution of n-butyllithium in hexane (23 mL, 32.66 mmol). The reaction mixture was warmed to room temperature and stirred during 1.5 h. Then the mixture was cooled to 0° C and potassium tertio-butylate (3.68 g, 32.8 mmol) and dry tertio-butyl alcohol (3mL, 31.8 mmol) were added. The mixture was stirred again at room temperature during 30 min. The reaction mixture was cooled to -78° C and the tetrahydrofuran solution of the aldehyde prepared above was added dropwise. The reaction mixture was warmed to room temperature and stirred during 2 h. A saturated aqueous solution of ammonium chloride and the solvents were evaporated under reduced pressure. The residue was dissolved in ether and washed with water. The organic layer was dried over sodium sulfate, filtered and concentrated under reduced pressure to afford a brown oil. Flash chromatography on silica gel and elution with a 4:96 mixture of ethyl acetate and toluene afforded the expected olefine methyl 2,3,4-tri-O-benzyl-6,7-dideoxy-α-D-glucohept-6-enopyranoside (3.26 g, 55%) which crystallized from hexane; m.p. 46-47° C.

## Example 5

Preparation of

METHYL 2,3,4-TRI-O-BENZYL-6-DEOXY-α-D-GLUCOHEPTOPYRANOSIDE

To a solution of methyl 2,3,4-tri-O-benzyl-6,7-dideoxy-α-D-glucohept-6-enopyranoside (0.878 g, 2.43 mmol) in dry tetrahydrofuran (5 mL) was added a 10 M solution of borane in methyl sulfide (0.24 mL, 2.4 mmol) at 0°C under nitrogen. The mixture was stirred during 3 h at room temperature. The excess of borane was destroyed with ethanol (1 mL). The mixture was cooled at 0°C. 30% hydrogen peroxyde (0.3 mL) were added. The mixture was refluxed during 2 h. The reaction mixture was diluted with water and extracted three times with ether. The organic layer was dried over sodium sulfate, filtered and concentrated under reduced pressure to afford an oil. Flash chromatography on silica gel and elution with a 1:1 mixture of ethyl acetate and hexane afforded the expected alcohol methyl 2,3,4-tri-O-benzyl-6-deoxy-α-D-glucoheptopranoside (0.414 g, 45%) which crystallized from hexane; m.p. 50-53°C.

## Example 6

Preparation of

METHYL 2,3,4-TRI-O-BENZYL-6-DEOXY-7-O-METHYLSULFONYL-α-D-GLUCOHEPTOPYRANOSIDE

To a solution of methyl 2,3,4-tri-O-benzyl-6-deoxy-α-D-glucoheptopyranoside (0.35 g, 0.92 mmol) in dry methylene chloride (10 mL) was added triethylamine (0.2 mL, 1.43 mmol). Then the solution was cooled to -10°C and mesylchloride (0.08 mL), 1 mmol) was added. The mixture was stirred an additional 15 min at -10°C, then the reaction was allowed to warm up to room temperature. The mixture was washed three times with water. The organic phase was dried over sodium sulfate, filtered and concentrated under reduced pressure to afford a yellow oil. Flash chromatography on silica gel and elution with a 40:60 mixture of ethylacetate and hexane afforded the expected mesylate methyl 2,3,4-tri-O-benzyl-6-deoxy-7-O-methylsulfonyl-α-D-glucoheptopyranoside as an oil (0.38 g, 91%).

## EXAMPLE 7

Preparation of

METHYL 2,3,4-TRI-O-BENZYL-6,7-DIDEOXY-7-IODO-α-D-GLUCOHEPTOPYRANOSIDE

To a solution of methyl 2,3,4-tri-O-benzyl-6-deoxy-7-O-methylsulfonyl-D-glucoheptopyranoside (0.38 g, 0.83 mmol) in ether (5 mL) was added at 0°C a 0.375 M solution of magnesium iodide (6.7 mL). The mixture was stirred 15 min at 0°C. The excess of magnesium iodide was hydrolyzed with water. The reaction mixture was washed with sodium, thiosulfate and water. The organic layer was dried over sodium sulfate, filtered and concentrated under reduced pressure to afford an oil. Flash chromatography on silica gel and elution with a 2:8 mixture of ethyl acetate and hexane afforded the expected iodide methyl 2,3,4-tri-O-benzyl-6,7-dideoxy-7-iodo-α-D-glucoheptopyranoside which was crystallized from hexane (0.368 g, 91%);

m.p. 66-68° C.

Example 8

Preparation of

2,3,5-TRI-O-BENZYL-1,4-DIDEOXY-1,4-[(2,3,4-TRI-O-BENZYL-6,7-DIDEOXY-1-O-METHYL-7-α-D-GLUCOHEPTOPYRANOSYL)IMINO]-L-ARABINITOL

A solution of methyl 2,3,4-tri-O-benzyl-6,7-dideoxy-7-iodo-α-D-glucoheptopyranoside (0.338 g, 0.69 mmol) and 2,3,5-tri-O-benzyl-1,4-dideoxy-1,4-imino-L-arabinitol (0.093 g, 0.23 mmol) in dry dimethylformamide (3 mL) is heated at 80° C overnight along with dry potassium carbonate (0.127 g, 0.92 mmol). The dimethylformamide is evaporated under reduced pressure. The residue is taken with ethyl acetate and washed twice with water. The organic layer is dried over sodium sulfate, filtered and concentrated under reduced pressure to afford an oil. Chromatography on neutral alumine activity III and elution with a 8:2 mixture of hexane and ethyl acetate will afford the expected amine 2,3,5-tri-O-benzyl-1,4-dideoxy-1,4-[-(2,3,4-tri-O-benzyl-6,7-dideoxy-1-O-methyl-7-α-D-glucoheptopyranosyl)imino]-L-arabinitol as a white foam.

Example 9

Preparation of

1,4-DIDEOXY-1,4-[(6, 7-DIDEOXY-1-O-METHYL-7-α-D-GLUCOHEPTOPYRANOSYL)IMINO]-L-ARABINITOL

2,3,5-tri-O-benzyl-1,4-dideoxy-1,4-[(2,3,4-tri-O-benzyl-6,7-dideoxy-1-O-methyl-7-α-D-glucoheptopyranosyl)imino]-L-arabinitol (0.1 g, 0.116 mmol) is dissolved in acetic acid (15 mL). Palladium 10% on charcoal (0.05 g) is added. The mixture is hydrogenated at 3 atmospheres during two days. The catalyst is removed by filtration and the solvents are evaporated under reduced pressure. The residue is dissolved in water and passed through a column of Amberlyst A26 $OH^{\ominus}$ form. Lyophilisation will afford 1,4-dideoxy-1,4-[(6,7-dideoxy-1-O-methyl-7-α-D-glucoheptopyranosyl)imino]-L-arabinitol.

Example 10

Preparation of

2,3,5-TRI-O-BENZYL-1,4-DIDEOXY-1,4-[(1-DEOXY-2,3:4,5-DI-O-ISOPROPYLIDENE-β-D-FRUCTOPYRANOSYL)IMINO]-L-ARABINITOL

A solution of 2,3:4,5-di-O-isopropylidene-1-O-trifluoromethylsulfonyl-β-D-fructopyranose (1.20 g, 3.06 mmol) (P.J. Card and W.D. Hitz, J. Amer. Chem. Soc., 106, 5348 (1984)) and 1,4-dideoxy-2,3,5,-tri-O-benzyl-1,4-imino-L-arabinitol (1.233 g, 3.06 mmol) in ethanol-free chloroform (70 ml) is refluxed under nitrogen during 60 h. The mixture is diluted with methylene chloride and washed successively with a saturated aqueous solution bicarbonate and saturated brine. The organic layer is dried over sodium sulfate,

filtered and concentrated under reduced pressure to afford an oil flash chromatography on silica gel and, elution with graded mixture of hexane and ethyl acetate, will afford the expected amine 2,3,5-tri-O-benzyl-1,4-dideoxy-1,4[(1-deoxy-2,3:4,5-di-O-isopropylidene-β-D-fructopyranosyl)imino]-L-arabinitol as an oil.

## Example 11

Preparation of

2,3,5-TRI-O-BENZYL-1,4-DIDEOXY-1,4-[(1-DEOXY-2-O-METHYL-α-D-FRUCTOFURANOSYL)IMINO]-L-ARABINITOL

2,3,5-tri-O-benzyl-1,4-dideoxy-1,4-[(1-deoxy-2,3:4,5-di-O-isopropylidene-β-D-fructopyranosyl)imino]-L-arabinitol (1.4 g, 2.17 mmol) is dissolved in methanol (100 mL) containing 2% of dry hydrochloric acid. The mixture is refluxed during 48 h. The mixture is neutralized with Amberlyst A26 $OH^{\ominus}$ form and filtered. The solvents are evaporated under reduced pressure. Flash chromatography on silica gel and, elution with graded mixture of ethyl acetate and methanol, will afford the expected amine 2,3,5-tri-O-benzyl-1,4-dideoxy-1,4-[(1-deoxy-2-O-methyl-α-D-fructofuranosyl)-imino]-L-arabinitol.

## Example 12

Preparation of

1,4-DIDEOXY-1,4-[(1-DEOXY-2-O-METHYL-α-D-FRUCTOFURANOSYL)IMINO]-L-ARABINITOL

The amine 2,3,5-tri-O-benzyl-1,4-dideoxy-1,4-[(1-deoxy-2-O-methyl-α-D-fructofuranosyl)imino]-L-arabinitol (0.617 g, 1.065 mmol) is dissolved in acetic acid (25 mL), palladium 10% on charcoal (0.3 g) is added. The mixture is hydrogenated during 3 days at 3 bars. The catalyst is removed by filtration and the solvents are evaporated under reduced pressure. The residue is dissolved in water and neutralized with Amberlyst A26 $OH^{\ominus}$ form and filtered. The mixture is put to dryness under reduced pressure. Flash chromatography on silica gel and elution, with graded mixture of chloroform, methanol and water, will afford the expected amine 1,4-dideoxy-1,4-[(deoxy-2-O-methyl-α-D-fructofuranosyl)imino]-L-arabinitol as an amorphous solid.

## Example 13

Preparation of

METHYL 2,3,6-TRI-O-BENZYL-4-O-TRIFLUOROMETHYLSULFONYL-α-D-GALACTOPYRANOSIDE

To a solution of dry pyridine (0.46 mL) in methylene chloride (17.5 mL) cooled to -15 °C is added trifluoromethane sulfonic anhydride (0.87 mL). The mixture is stirred during 15 min at -10 °C, then methyl 2,3,6-tri-O-benzyl-α-D-galactopyranoside (1.2 g, 2.58 mmol) in methylene chloride (5 mL) is added (N. Morishima, S. Koto, M. Oshima, A. Sugimoto and S. Zen, Bull. Chem. Soc. Jpn, 56, 2849 (1983)). The

mixture is washed with water. The organic layer is dried over sodium sulfate, filtered and concentrated under reduced pressure to afford an oil which will be the expected triflate methyl 2,3,6-tri-O-benzyl-4-O-trifluoromethyl-sulfonyl-α-D-galactopyranoside.

Example 14

Preparation of

2,3,5-TRI-O-BENZYL-1,4-DIDEOXY-1,4-[(2,3,6-TRI-O-BENZYL-4-DEOXY-1-O-METHYL-4-α-D-GLUCOPYRANOSYL)IMINO]-L-ARABINITOL

A solution of methyl 2,3,6-tri-O-benzyl-4-O-trifluoromethyfulfonyl-α-D-galactopyranoside (1.25 g, 2.53 mmol) and 2,3,5-tri-O-benzyl-1,4-dideoxy-1,4-imino-L-arabinitol (1.019 g, 2.53 mmol) in ethanol-free chloroform (70 mL) is refluxed under nitrogen during 3 days. The mixture is diluted with methylene chloride and washed successively with a saturated aqueous solution of sodium bicarbonate and saturated brine. The organic layer is dried over sodium sulfate, filtered and concentrated under reduced pressure to afford an oil. Flash chromatography on silica gel and, elution with graded mixture of hexane and ethyl acetate, will afford the expected amine 2,3,5-tri-O-benzyl-1,4-dideoxy-1,4-[(2,3,6-tri-O-benzyl-4-deoxy-1-O-methyl-4-α-D-glucopyranosyl)imino]-L-arabinitol as an oil.

Example 15

Preparation of

1,4-DIDEOXY-1,4-[(4-DEOXY-1-O-METHYL-4-α-D-GLUCOPYRANOSYL)-IMINO]-L-ARABINITOL

The amine 2,3,5-tri-O-benzyl-1,4-dideoxy-1,4-[(2,3,6-tri-O-benzyl-4-deoxy-1-O-methyl-α-D-glucopyranosyl)imino]-L-arabinitol (0.91 g, 1.07 mmol) is dissolved in acetic acid (20 mL). Palladium 10% on charcoal (0.5g) is added. The mixture is hydrogenated during 4 days at 3 bars. The catalyst is filtered. The solvents are evaporated under reduced pressure. The residue is dissolved in water and neutralized with Amberlyst A26 $OH^{\ominus}$ form. The mixture is filtered and the aqueous layer is put to dryness under reduced pressure to afford a foam. Flash chromatography on silica gel and, elution with a 50:50:4 mixture of methanol, chloroform and water, will afford the expected amine 1,4-dideoxy-1,4-[(4-deoxy-1-O-methyl-4-α-D-glucopyranosyl)imino]-L-arabinitol as a foam.

Example 16

Preparation of

METHYL 2,3,4-TRI-O-BENZYL-6-O-(2,3,4-TRI-O-BENZYL-6-O-TRIFLUOROMETHYLSULFONYL-α-D-GLUCOPYRANOSYL-α-D-GLUCOPYRANOSIDE

To a solution of dry pyridine (0.24 mL) in methylene chloride (25 mL) cooled to -15°C was added

trifluoromethane sulfonic anhydride (0.45 mL). The mixture was stirred during 15 min at -10° C, then methyl 6-O-(2,3,4-tri-O-benzyl-α-D-gluco pyranosyl)-2,3,4-tri-O-benzyl-α-D-glucopyranoside (1.2 g, 1.34 mmol) in methylene chloride (5 mL) is added (R. Eby and C. Schuerch, Carbohydr. Res., 50, 203 (1976)). The mixture is stirred during 1.5 h at -10° C. The reaction mixture is washed with water. The organic layer is dried over sodium sulfate, filtered and concentrated under reduced pressure to afford an oil (1.35 g, 98%) which will be the expected triflate methyl 2,3,4-tri-O-benzyl-6-O-(2,3,4-tri-O-benzyl-6-O-trifluoromethylsulfonyl-α-D-glucopyranosyl)-α-D-glucopyranoside.

## Example 17

Preparation of

2,3,5-TRI-O-BENZYL-1,4-DIDEOXY-N-[2,3,4-TRI-O-BENZYL-6-DEOXY-1-(2,3,4-TRI-O-BENZYL-1-O-METHYL-6-O-α-D-GLUCOPYRANOSYL)-α-D-GLUCOPYRANOSYL]-1,4-IMINO-L-ARABINITOL

A solution of methyl 2,3,4-tri-O-benzyl-6-O-(2,3,4-tri-O-benzyl-6-O-trifluoromethysulfonyl-α-D-glucopyranosyl)-α-D-glucopyranoside (1.35 g, 1.31 mmol) and 2,3,5-tri-O-benzyl-1,4-dideoxy-1,4-imino-L-arabinitol (0.528 g, 1.31 mmol) in ethanolfree chloroform (50 mL) is refluxed under nitrogen during 48 h. The mixture is diluted with methylene chloride and washed successively with a saturated aqueous solution of sodium bicarbonate and saturated brine. The organic layer is dried over sodium sulfate, filtered and concentrated under reduced pressure to afford a foam. Flash chromatography on silica gel and, elution with graded mixture of hexane and ethyl acetate, will afford the expected amine 2,3,5-tri-O-benzyl-1,4-dideoxy-N-[2,3,4-tri-O-benzyl-6-deoxy-1-(2,3,4-tri-O-benzyl-1-O-benzyl-1-O-methyl-6-O-α-D-glucopyranosyl)-α-D-glucopyranosyl]-1,4-imino-L-arabinitol.

## Example 18

Preparation of

1,4-DIDEOXY-N-[6-DEOXY-1-(1-O-METHYL-6-O-α-D-GLUCOPYRANOSYL)-α-D-GLUCOPYRANOSYL]-1,4-IMINO-L-ARABINITOL

The amine 2,3,5-tri-O-benzyl-1,4-dideoxy-N-[2,3,4-tri-O-benzyl-6-deoxy-1-(2,3,4-tri-O-benzyl-1-O-methyl-6-O-α-D-glucopyranosyl)-α-D-glucopyranosyl]-1,4-imino-L-arabinitol (1.2 g, 0.937 mmol) is dissolved in methanol (30 mL). Palladium hydroxyde 20% on charcoal (0.5 g) is added. The mixture is hydrogenated during 4 days at 3 atmosphere. The catalysor is removed by filtration and the solvents are evaporated under reduced pressure. Flash chromatography on silica gel and elution with graded mixture of chloroform, methanol and water will afford the expected amine 1,4-dideoxy-N-[6-deoxy-1-(1-O-methyl-6-O-α-D-glucopyranosyl)-α-D-glucopyranosyl]-1,4-imino-L-arabinitol.

## Example 19

Preparation of

METHYL 6-O-(2,3,4-TRI-O-BENZYL-6,7-DIDEOXY-α-D-GLUCOHEPT-6-ENOPYRANOSYL)-2,3,4-TRI-O-BENZYL-α-D-GLUCOPYRANOSIDE

To a solution of oxalyl chloride (0.37 mL, 5.97 mmol) in dry tetrahydrofuran (40 mL) cooled to -78° C, dry dimethyl sulfoxyde (0.45 mL, 6.26 mmol) is added dropwise and then stirred during 35 min at -35° C. The reaction mixture is cooled again to -78° C and methyl 6-O-(2,3,4-tri-O-benzyl-α-D-glucopyranosyl)-2,3,4-tri-O-benzyl-α-D-glucopyranoside (5.1 g, 5.69 mmol) dissolved in tetrahydrofuran (20 mL) is added and the mixture is stirred during 15 min at -35° C, then triethylamine (3.96 mL, 28.45 mmol) is added and the mixture is stirred during 1 h at -35° C. This aldehyde is used without purification and isolation in a Wittig reaction described as follows. To dried triphenylmethylphosphonium bromide (4.059 g, 11.38 mmol) suspended in tetrahydrofuran (100 mL) is added dropwise at -78° C a 1.55 M solution of n-butyllithium in hexane (7.34 mL, 11.38 mmol). The reaction mixture is warmed to room temperature and stirred during 1.5 h. Then the mixture is cooled to 0° C and potassium tertio-butylate (1.275 g, 11.38 mmol) and dry tertio-butyl alcohol (1.04 mL, 11.38 mmol) are added. The mixture is stirred again at room temperature during 30 min. The reaction mixture is cooled to -78° C and the tetrahydrofuran solution of the aldehyde prepared above is added dropwise. The reaction mixture is warmed to room temperature and stirred during 2 h. A saturated aqueous solution of ammonium chloride and the solvents are evaporated under reduced pressure. The residue is dissolved in ethyl acetate and washed with water. The organic layer is dried over sodium sulfate, filtered and concentrated under reduced pressure. Flash chromatography on silica gel and elution with a graded mixture of carbon tetrachloride and ethyl acetate will afford the expected olefine methyl 6-O-(2,3,4-tri-O-benzyl-6,7-dideoxy-α-D-glucohept-6-enopyranosyl)-2,3,4-tri-O-benzyl-α-D-glucopyranoside.

## Example 20

Preparation of

METHYL 6-O-(2,3,4-TRI-O-BENZYL-6-DEOXY-α-D-GLUCOHEPTOPYRANOSYL)-2,3,4-TRI-O-BENZYL-α-D-GLUCOPYRANOSIDE

To a solution of methyl 6-O-(2,3,4-tri-O-benzyl-6,7-dideoxy-α-D-glucohept-6-enopyranosyl)-2,3,4-tri-O-benzyl-α-D-glucopyranoside (2.54 g, 2.85 mmol) in dry tetrahydrofuran (10 mL) is added a 10 M solution of borane in methyl sulfide (0.28 mL, 2.8 mmol) at 0° C under nitrogen. The mixture is stirred during 3 h at room temperature. Then the mixture is cooled to 0° C. The excess of borane is destroyed with ethanol (1 mL). The mixture is cooled at 0° C. 30% hydrogen peroxyde (0.3 mL) and 3 N aqueous solution of sodium hydroxyde (0.3 mL) are added. The mixture is refluxed during 2 h. The reaction mixture is diluted with water and extracted three times with ethyl acetate. The organic layer is dried over sodium sulfate, filtered and concentrated under reduced pressure. Flash chromatography on silica gel and elution with a graded mixture of carbon tetrachloride and ethyl acetate will afford the expected alcohol methyl 6-O-(2,3,4-tri-O-benzyl-6-deoxy-α-D-glucoheptopyranosyl)-2,3,4-tri-O-benzyl-α-D-glucopyranoside.

## Example 21

Preparation of

METHYL 6-O-(2,3,4-TRI-O-BENZYL-6,7-DIDEOXY-7-IODO-α-D-GLUCOHEPTOPYRANOSYL)-2,3,4-TRI-O-BENZYL-α-D-GLUCOPYRANOSIDE

To a solution of methyl 6-O-(2,3,4-tri-O-benzyl-6-deoxy-α-D-glucoheptopyranosyl)-2,3,4-tri-O-benzyl-α-D-glucopyranoside (1.245 g, 1.37 mmol) in dry methylene chloride (15 mL) is added triethylamine (0.29 mL, 2.05 mmol). Then the solution is cooled to -10° C, and mesylchloride (0.11 mL, 1.42 mmol) is added dropwise. The mixture is stirred an additional 15 min at -10° C, then the reaction mixture is washed three times with water. The organic layer is dried over sodium sulfate, filtered and concentrated under reduced pressure to afford a foam which is used without further purification. The crude methyl 6-O-(2,3,4-tri-O-benzyl-6-deoxy-7-O-methylsulfonyl-α-D-glucoheptopyranosyl)-2,3,4-tri-O-benzyL-α-D-glucopyranoside is dissolved in ether (20 mL). To this mixture a 0.35 M solution of magnesium iodide in ether (17.5 mL) is added dropwise at 0° C. The excess of magnesium iodide is hydrolyzed with water. The reaction mixture is washed with sodium, thiosulfate and water. The organic layer is dried over sodium sulfate, filtered and concentrated under reduced pressure to afford a foam. Flash chromatography on silica gel and elution with a graded mixture of carbon tetrachloride and ethyl acetate will afford the expected iodide methyl 6-O-(2,3,4-tri-O-benzyl-6,7-dideoxy-7-iodo-α-D-glucoheptopyranosyl)-2,3,4-tri-O-benzyl-α-D-glucopyranoside.

## Example 22

Preparation of

2,3,5-TRI-O-BENZYL-1,4-DIDEOXY-N-[2,3,4-TRI-O-BENZYL-6,7-DIDEOXY-1-(2,3,4-TRI-O-BENZYL-1-O-METHYL-6-O-α-D-GLUCOPYRANOSYL)-α-D-GLUCOHEPTOPYRANOSYL]-1,4-IMINO-L-ARABINITOL

A solution of the iodide methyl 6-O-(2,3,4-tri-O-benzyl-6,7-dideoxy-7-iodo-α-D-glucoheptopyranosyl)-2,3,4-tri-O-benzyl-α-D-glucopyranoside (1.145 g, 1.122 mmol) and the amine 2,3,5-tri-O-benzyl-1,4-dideoxy-1,4-imino-L-arabinitol (0.150 g, 0.374 mmol) in dry dimethlformamide (4mL) is heated at 80° C overnight along with dry potassium carbonate (0.206 g, 1.49 mmol). The dimethylformamide is evaporated under reduced pressure. The residue is taken with ethyl acetate and washed twice with water. The organic layer is dried over sodium sulfate, filtered and concentrated under reduced pressure to afford a foam. Chromatography on neutral aluminum oxyde activity III and elution with a graded mixture of carbon tetrachloride and ethyl acetate will afford the expected amine 2,3,5-tri-O-benzyl-1,4-dideoxy-N-[2,3,4-tri-O-benzyl-6,7-dideoxy-1-(2,3,4-tri-O-benzyl-1-O-methyl-6-O-α-D-glucopyranosyl)-α-D-glucoheptopyranosyl]-1,4-imino-L-arabinitol.

## Example 23

Preparation of

1,4-DIDEOXY-N-[6,7-DIDEOXY-1-(1-O-METHYL-6-O-α-D-GLUCOPYRANOSYL)-α-D-GLUCOHEPTOPYRANOSYL]-1,4-IMINO-L-ARABINITOL

The amine 2,3,5-tri-O-benzyl-1,4-dideoxy-N-[2,3,4-tri-0benzyl-6,7-dideoxy-1-(2,3,4-tri-O-benzyl-1-O-methyl-6-O-α-D-glucopyranosyl)-α-D-glucoheptopyranosyl]-1,4-imino-L-arabinitol (0.329 g, 0.254 mmol) is dissolved in acetic acid (30 mL). Palladium 10% on charcoal (0.4 g) is added. The mixture is hydrogenated during 4 days at 3 atmospheres. The catalyst is removed by filtration and the solvents are evaporated under reduced pressure. The residue is dissolved in water and passed through a column of Amberlyst A26 $OH^{\ominus}$. Water is evaporated under reduced pressure. Flash chromatography on silica gel and elution with a graded mixture of chloroform, methanol and water will afford the expected amine 1,4-dideoxy-N-[6,7-dideoxy-1-(1-O-methyL-6-O-α-D-glucopyranosyl)-α-D-glucoheptopyranosyl]-1,4-imino-L-arabinitol.

## Example 24

Preparation of

METHYL 2,3,6-TRI-O-BENZYL-4-CYANO-4-DEOXY-α-D-GLUCOPYRANOSIDE

A solution of methyl 2,3,6-tri-O-benzyl-4-O-trifluoromethylsulfonyl-α-D-galactopyranoside (3 g, 6.07 mmol) and tetra-n-butyl ammonium cyanide (6.51 g, 24.28 mmol) in ethanol-free chloroform (60 mL) is refluxed under nitrogen during 24 h. The reaction mixture is diluted with methylene chloride, washed twice with water. The organic layer is dried over sodium sulfate, filtered and concentrated under reduced pressure to afford an oil. Flash chromatography on silica gel and elution with a graded mixture of hexane and ethyl acetate will afford the expected nitrile methyl 2,3,6-tri-O-benzyl-4-cyano-4-deoxy-α-D-glucopyranoside.

Example 25

Preparation of

METHYL 2,3,6-TRI-O-BENZYL-4-DEOXY-4-FORMYL-α-D-GLUCO-PYRANOSIDE

To a solution of methyl 2,3,6-tri-O-benzyl-4-cyano-4-deoxy-α-D-glucopyranoside (1.75 g, 3.7 mmol) in dry tetrahydrofuran (10 mL) is added dropwise at -78 °C a 1.2 M solution of diisobutyl aluminum hydride in n-hexane (3.1 mL). The mixture is stirred under argon at -78 °C during 3 h. Methanol (2 mL) is added and the mixture is warmed to 0 °C. Then the solvents are evaporated under reduced pressure. Ether (50 mL) and 0.1 N aqueous hydrochloric acid (40 mL) are added, the mixture is stirred at 0 °C during 1 h. Then after decantation the organic layer is dried over sodium sulfate, filtered and concentrated under reduced pressure to afford the expected aldehyde methyl 2,3,6-tri-O-benzyl-4-deoxy-4-formyl-α-D-glucopyranoside which is used without purification.

Example 26

Preparation of

METHYL 2,3,6-TRI-O-BENZYL-4-DEOXY-4-HYDROXYMETHYL-α-D-GLUCOPYRANOSIDE

The aldehyde methyl 2,3,6-tri-O-benzyl-4-deoxy-4-formyl-α-D-glucopyranoside (1.7 g, 3.57 mmol) is dissolved in ethanol (15 mL). The mixture is cooled to 0 °C and solid sodium borohydride (0.068g, 1.8 mmol) is added portionwise. The mixture is stirred 1 h at 0 °C. Then acetic acid (0.4 mL) is added and the solvents are evaporated under reduced pressure. The residue is taken with ethyl acetate and washed with saturated aqueous sodium bicarbonate and saturated brine. The organic layer is dried over sodium sulfate, filtered and concentrated under reduced pressure to afford an oil. Flash chromatography over silica gel and elution with a graded mixture of hexane and ethyl acetate will afford the expected alcohol methyl 2,3,6-tri-O-benzyl-4-deoxy-4-hydroxy-methyl-α-D-glucopyranoside.

Example 27

Preparation of

METHYL 2,3,6-TRI-O-BENZYL-4-DEOXY-4-TRIFLUOROMETHYLSULFONYLOXYMETHYL-α-D-GLUCOPYRANOSIDE

To a solution of dry pyridine (0.45 mL) in methylene chloride (30 mL) cooled to -15° C is added trifluoromethanesulfonic anhydride (0.84 mL). The mixture is stirred during min at -10° C, then methyl 2,3,6-tri-O-benzyl-4-deoxy-4-hydroxymethyl-α-D-glucopyranoside (1.19 g, 2.49 mmol) in methylene chloride (5 mL) is added. The mixture is stirred during 1.5 h at -10° C. The reaction mixture is washed with water. The organic layer is dried over sodium sulfate, filtered and concentrated under reduced pressure to afford an oil (1.443 g, 95%) which will be the expected triflate methyl 2,3,6-tri-O-benzyl-4-deoxy-4-trifluoromethylsulfonyloxymethyl-α-D-glucopyranoside.

Example 28

Preparation of

2,3,5-TRI-O-BENZYL-1,4-DIDEOXY-1,4-[(2,3,6-TRI-O-BENZYL-4-DEOXY-1-O-METHYL-4-α-D-GLUCOPYRANOSYL)METHYLIMINO]-L-ARABINITOL

A solution of methyl 2,3,6-tri-O-benzyl-4-deoxy-4-trifluoromethylsulfonyloxymethyl-α-D-glucopyranoside (1 g, 1.64 mmol) and 2,3,5-tri-O-benzyl-1,4-dideoxy-1,4-imino-L-arabinitol (0.66 g, 1.64 mmol) in ethanol-free chloroform (60 mL) is refluxed under nitrogen during 48 h. The mixture is diluted with methylene chloride and washed successively with a saturated aqueous solution of sodium bicarbonate and saturated brine. The organic layer is dried over sodium sulfate, filtered and concentrated under reduced pressure to afford a foam. Flash chromatography on silica gel and elution with a graded mixture of hexane and ethyl acetate will afford the expected amine 2,3,5-tri-O-benzyl-1,4-dideoxy-1,4-[(2,3,6-tri-O-benzyl-4-deoxy-1-O-methyl-4-α-D-glucopyranosyl)methylimino]-L-arabinitol.

Example 29

Preparation of

1,4-DIDEOXY-1,4-[(4-DEOXY-1-O-METHYL-4-α-D-GLUCOPYRANOSYL)METHYLIMINO]-L-ARABINITOL

The amine 2,3,6-tri-O-benzyl-1,5-dideoxy-1,5-[(2,3,6-tri-O-benzyl-4-deoxy-1-O-methyl-4-α-D-glucopyranosyl)methyl-imino]-L-arabinitol (0.98 g, 1.13 mmol) is dissolved in methanol (20 mL). Cyclohexene (10 mL) and palladium hydroxyde 20% on charcoal (0.8 g) are added and the mixture is refluxed under nitrogen during 8 h. The catalyst is removed by filtration and the solvents are evaporated under reduced pressure. Flash chromatography on silica gel and elution with a graded mixture of chloroform, methanol and water will afford the expected amine 1,4-dideoxy-1,4-[(4-deoxy-1-O-methyl-4-α-D-glucopyranosyl)-methylimino]-L-arabinitol.

Example 30

Preparation of

2,3,6-TRI-O-BENZYL-D-GALACTOPYRANOSE

Methyl 2,3,6-tri-O-benzyl-α-D-galactopyranoside (5 g, 10.775 mmol) is dissolved at 0° C in a 9:1 mixture of trifluoroacetic acid and water (50 mL) (N.Morishima, S.Koto, M.Oshima, A.Sugimoto and S.Zen, Bull Chem. Soc. Jpn 56, 2849 (1983)). The mixture is stirred overnight at 0° C. The solvents are evaporated under reduced pressure without heating. The residue is dissolved in ethyl acetate and washed successively with sodium bi-carbonate and brine. The organic layer is dried over sodium sulfate, filtered and concentrated under reduced pressure to afford an oil. Flash chromatography on silica gel and elution with a graded mixture of ethyl acetate and hexane will afford 2,3,6-tri-O-benzyl-D-galactopyranose.

Example 31

Preparation of

1,4-DI-O-ACETYL-2,3,6-TRI-O-BENZYL-D-GALACTOPYRANOSE

2,3,6-tri-O-benzyl-D-galactopyranose (3.927 g, 8.72 mmol) is dissolved in dry pyridine (25 mL) and acetic anhydride (5 mL) is added. The mixture is stirred during 24 h at room temperature. The solvent is evaporated under high vacuum. The residue is dissolved in ethyl acetate and washed with water. The organic layer is dried over sodium sulfate, filtered and concentrated under reduced pressure and will afford the expected diacetate 1,4-di-O-acetyl-2,3,6-tri-O-benzyl-D-galactopyranose as an oil usable without purification.

Example 32

Preparation of

4-O-ACETYL-2,3,6-TRI-O-BENZYL-α-D-GALACTOPYRANOSYL CHLORIDE

A solution of 1,4-di-O-acetyl-2,3,6-tri-O-benzyl-D-galactopyranose (4.64 g, 8.67 mmol) in ether (10 mL) is treated with ethereal hydrogen chloride (0.2 g/mL, 25 mL). The mixture is stirred at room temperature during 48 h. The solvents are evaporated under reduced pressure to afford an oil. Flash chromatography on silica gel and elution with a graded mixture of carbon tetrachloride and ethyl acetate will afford 4-O-acetyl-2,3,6-tri-O-benzyl-α-D-galactopyranosyl chloride (3.142 g, 71%) as an oil.

Example 33

Preparation of

METHYL 4-O-(4-O-ACETYL-2,3,6-TRI-O-BENZYL-α-D-GALACTOPYRANOSYL)-2,3,6-TRI-O-BENZYL-α-D-GLUCOPYRANOSIDE

Ethereal silver perchlorate (0.08 M, 84.5 mL, 6.76 mmol) is added with stirring at -30 °C to a solution of methyl-2,3,6-tri-O-benzyl-a-D-glucopyranoside (2.284 g, 4.93 mmol) [P.J. Garegg, H. Hultberg and S. Wallin, Carbohydr. Res., 108, 97 (1982)], 4-O-acetyL-2,3,6-tri-O-benzyl-α-D-galactopyranosyl chloride (3.142 g, 6.154 mmol) and 2,4,6-trimethylpyridine (0.89 mL, 6.76 mmol) in ether (20 mL). The mixture is stirred min at -30 °C and silver chloride precipitates. The mixture is filtered through a celite pad, the solids are washed with ether, the filtrate is concentrated under reduced pressure. The residue is dissolved in methylene chloride and the organic layer is washed successively with aqueous sodium thiosulfate and water. The organic layer is dried over sodium sulfate, filtered and concentrated under reduced pressure to afford a foam. Flash chromatography on silica gel and elution with a graded mixture of hexane and ethyl acetate will afford methyl 4-O-(4-O-acetyl-2,3,6-tri-O-benzyl-α-D-galactopyranosyl)1,3,6-tri-O-benzyl-α-D-glucopyranoside.

## Example 34

Preparation of

METHYL 2,3,6-TRI-O-BENZYL-4-O-(2,3,6-TRI-O-BENZYL-α-D-GALACTOPYRANOSYL)-α-D-GLUCOPYRANOSIDE

Methyl 4-O-(4-O-acetyl-2,3,6-tri-O-benzyL-α-D-galactopyranosyl)-2,3,6-tri-O-benzyl-α-D-glucopyranoside (2.543 g, 2.71 mmol) is dissolved in hot toluene (20 mL) and methanol (80 mL) is added, followed by a few drops of 1 M. methanolic sodium methoxide. The mixture is stirred at room temperature during 2 h. The reaction mixture is made neutral with Amberlite IR 120 (H+) resin, filtered and concentrated under reduced pressure and will afford methyl 2,3,6-tri-O-benzyl-4-O-(2,3,6-tri-O-benzyl-α-D-galactopyranosyl)-α-D-glucopyranoside (2.42 g, 100%) as an amorphous solid.

## Example 35

Preparation of

METHYL 4-O-(2,3,6-TRI-O-BENZYL-4-O-TRIFLUOROMETHYLSULFONYL-α-D-GALACTOPYRANOSYL)-2,3,6-TRI-O-BENZYL-α-D-GLUCOPYRANOSIDE

To a solution of dry pyridine (0.49 mL) in dry methylene chloride (40 mL) cooled to -15 °C is added trifluoromethanesulfonic anhydride (0.91 mL). The mixture is stirred during 15 min at -10 °C, then methyl 2,3,6-tri-O-benzyl-4-O-(2,3,6-tri- O-benzyl-α-D-galactopyranosyl)-α-D-glucopyranoside (2.428 g, 2.71 mmol) in methylene chloride (10 mL) is added. The mixture is stirred during 1.5 h at -10 °C. The reaction mixture is washed with water. The organic layer is dried over sodium sulfate, filtered and concentrated under reduced pressure to afford an oil which will be the expected triflate methyl 4-O-(2,3,6-tri-O-benzyl-4-O-trifluoromethylsulfonyl-α-D-galactopyranosyl)-2,3,6-tri-O-benzyl-α-D-glucopyranoside.

## Example 36

Preparation of

2,3,5-TRI-O-BENZYL-1,4-DIDEOXY-N-[2,3,6-TRI-O-BENZYL-4-DEOXY-1-(2,3,6-TRI-O-BENXYL-1-O-METHYL-4-O-α-D-GLUCOPYRANOSYL)-α-D-GLUCOPYRANOSYL]-1-4-IMINO-L-ARABINITOL

A solution of methyl 4-O-(2,3,6-tri-O-benzyl-4-O-tri-fluoromethysulfonyl-α-D-galactopyranosyl)-2,3,6-tri-O-benzyl-α-D-glucopyranoside (1.52 g, 1.46 mmol) and 2,3,5-tri-O-benzyl-1,4-dideoxy-1,4-imino-L-arabinitol (0.588 g, 1.46 mmol) in ethanol-free chloroform (50 mL) is refluxed under nitrogen during 48 h. The mixture is diluted in methylene chloride and washed successively with a saturated aqueous solution of sodium bicarbonate and saturated brine. The organic layer is dried over sodium sulfate, filtered and concentrated under reduced pressure to afford a foam. Flash chromatography on silica gel and elution with a graded mixture of hexane and ethyl acetate will afford the expected amine 2,3,5-tri-O-benzyl-1,4-dideoxy-N-[2,3,6-tri-O-benzyl-4-deoxy-1-(2,3,6-tri-O-benzyl-1-O-methyl-4-O-α-D-gluco-pyranosyl)-α-D-glucopyranosyl]-1,4-imino-L-arabinitol.

## Example 37

Preparation of

1,4-DIDEOXY-N-[4-DEOXY-1-(1-O-METHYL-4-O-α-D-GLUCOPYRANOSYL)-α-D-GLUCOPYRANOSYL]-1,4-IMINO-L-ARABINITOL

2,3,5-tri-O-benzyl-1,4-dideoxy-N-[2,3,6-tri-O-benzyl-4-deoxy-1-(2,3,6-tri-O-benzyl-1-methyl-4-O-α-D-glucopyranosyl)-α-D-glucopyranosyl]-1,4-imino-L-arabinitol (1 g, 0.78 mmol) is dissolved in acetic acid (30 mL). Palladium 10% on charcoal (0.5 g) is added. The mixture is hydrogenated during 4 days at 3 atmospheres. The catalyst is removed by filtration and the solvents are evaporated under reduced pressure. The residue is taken with water and passed through a column of Amberlyst A26 $OH^{\ominus}$ form. Water is evaporated under reduced pressure and flash chromatography on silica gel and elution with a graded mixture of chloroform, methanol and water will afford the expected amine 1,4-dideoxy-N-[4-deoxy-1-(1-O-methyl-4-O-α-D-glucopyranosyl)-α-D-glucopyranosyl]-1,4-imino-L-arabinitol.

## Example 38

Preparation of

1-ETHENYL-1,2:3,4-DI-O-ISOPROPYLIDENE-β-D-ARABINOPYRANOSE

To a solution of oxalyl chloride (1.05 mL, 17.22 mmol) in dry tetrahydrofuran (40 mL) cooled to -78°C, dry dimethyl sulfoxyde (1.3 mL, 18.04 mmol) is added dropwise and then stirred during 35 min at -35°C. The reaction mixture is cooled again to -78°C and 2,3:4,5-di-O-isopropylidene-D-fructopyranose (4.26 g, 16.4 mmol) (R.F. Brady, Carbohydr. Res., 15, 35 (1970)) dissolved in tetrahydrofuran (20 mL) is added and the mixture is stirred during 15 min at -35°C, then triethylamine (11.5 mL, 82.65 mmol) is added and the

mixture is stirred during 1 h at -35 °C. This aldehyde is used without purification and isolation in a Wittig reaction described as follows. To dried triphenyl-methylphosphonium bromide (11.7 g, 32.8 mmol) suspended in tetrahydrofuran (400 mL) is added dropwise at -78 °C a 1.55 M solution of n-butyllithium in hexane (21 mL, 32.66 mmol). The reaction mixture is warmed to room temperature and stirred during 1.5 h. Then the mixture is cooled to 0 °C and potassium tertio-butylate (3.68 g, 32.8 mmol) and dry tertiobutyl alcohol (3 mL, 31.8 mmol) are added. The mixture is stirred again at room temperature during 30 min. The reaction mixture is cooled to -78 °C and the tetrahydrofuran solution of the aldehyde prepared above is added dropwise. The reaction mixture is warmed to room temperature and stirred during 2 h. A saturated aqueous solution of ammonium chloride and the solvents are evaporated under reduced pressure. The residue is dissolved in ether and washed with water. The organic layer is dried over sodium sulfate, filtered and concentrated under reduced pressure to afford a brown oil. Flash chromatography on silica gel and elution with a graded mixture of hexane and ethyl acetate will afford the expected olefine 1-ethenyl-1,2:3,4-di-O-isopropylidene-β-D-arabinopyranose.

## Example 39

Preparation of

1,2:3,4-DI-O-ISOPROPYLIDENE-1-(2-HYDROXYETHYL)-β-D-ARABINOPYRANOSE

To a solution of 1-ethenyl-1,2:3,4-di-O-isopropylidene-β-D-arabinopyranose (2 g, 7.81 mmol) in dry tetrahydrofuran (15 mL) is added a 10 M solution of borane in methyl sulfide (0.78 mL, 7.8 mmol) at 0 °C under nitrogen. The mixture is stirred during 3 h at room temperature. The excess of borane is destroyed with ethanol (3 mL). The mixture is cooled at 0 °C. 30% hydrogen peroxyde (1 mL) and 3 N aqueous solution of sodium hydroxyde (1 mL) are added. The mixture is refluxed during 2 h. The reaction mixture is diluted with water and extracted three times with ethyl acetate. The organic layer is dried over sodium sulfate, filtered and concentrated under reduced pressure to afford an oil. Flash chromatography on silica gel and elution with a 1:1 mixture of ethyl acetate and hexane will afford the expected alcohol 1,2:3,4-di-O-isopropylidene-1-(2-hydroxyethyl)-β-D-arabinopyranose.

## Example 40

Preparation of

1,2:3,4-DI-O-ISOPROPYLIDENE-1-(2-IODOETHYL)-β-D-ARABINOPYRANOSE

To a solution of 1,2:3,4-di-O-isopropylidene-1-(2-hydroxyethyl)-β-D-arabinose (1.7 g, 6.2 mmol) in dry methylene chloride (30 mL) is added triethylamine (1.3 mL, 9.3 mmol). Then the mixture is cooled to -10 °C and mesylchloride (0.5 mL, 6.46 mmol) is added dropwise. The mixture is stirred an additional 15 min at -10 °C, then the reaction is allowed to warm up to room temperature. The mixture is washed three times with water. The organic phase is dried over sodium sulfate, filtered and concentrated under reduced pressure to afford a yellow oil which is used without purification. The crude 1,2:3,4-di-O-isopropylidene-1-(2-methylsulfonyloxyethyl)-α-D-arabinose is dissolved in ether (15 mL). To this mixture a 0.35 M solution of magnesium iodide in ether (53 mL) is added at 0 °C. The mixture is stirred 15 min at 0 °C. The excess of magnesium iodide is hydrolyzed with water. The reaction mixture is washed with aqueous sodium thiosulfate and water. The organic layer is dried over sodium sulfate, filtered and concentrated under reduced pressure to afford an oil. Flash chromatography on silica gel and elution with a 9:1 mixture of hexane and ethylacetate will afford the expected iodide 1,2:3,4-di-O-isopropylidene-1-(2-iodoethyl)-β-D-arabinopyranose.

Example 41

Preparation of

2,3,5-TRI-O-BENZYL-1,4-DIDEOXY-1,4-{[2-(1,2:3,3-DI-O-ISOPROPYLIDENE-1-β-D-ARABINOPYRANOSYL)-ETHYL]IMINO}-L-ARABINITOL

A solution of 1,2:3,4-di-O-isopropylidene-1-(2-iodoethyl)β-D-arabinose (1.9 g, 4.95 mmol) and 2,3,5-tri-O-benzyl-1,4-dideoxy-1,4-imino-L-arabinitol (0.665 g, 1.65 mmol) in dry dimethylformamide (10 mL) is heated at 80° C overnight along with dry potassium carbonate (0.91 g, 6.6 mmol). The dimethylformamide is evaporated under reduced pressure. The residue is taken with ethyl acetate and washed twice with water. The organic layer is dried over sodium sulfate, filtered and concentrated under reduced pressure to afford an oil. Chromatography on neutral aluminum oxyde activity III and elution with a graded mixture of hexane and ethyl acetate will afford the expected amine 2,3,5-tri-O-benzyl-1,4-dideoxy-1,4{[2-(1,2:3,4-di-O-isopropylidene-1-β-D-arabinopyranosyl)] imino}-L-arabinitol.

Example 42

Preparation of

2,3,5-TRI-O-BENZYL-1,4-DIDEOXY-1,4-{[2-(1-O-METHYL-1-α-D-ARABINOFURANOSYL)ETHYL]IMINO}-L-ARABINITOL

2,3,5-tri-O-benzyl-1,4-dideoxy-1,4-{[2-(1,2:3,4-di-O-isopropylidene-1-β-D-arabinopyranosyl)ethyl]-imino}-L-arabinitol (0.74 g, 0.95 mmol) is dissolved in methanol (60 mL) containing 5% of dry hydrochloric acid and is refluxed during 24 h. The reaction mixture is cooled to room temperature and neutralized with Amberlyst A26 $OH^-$ form. The mixture is filtered and the solvent is evaporated under reduced pressure to give a foam. Flash chromatography on silica gel and elution with a graded mixture of ethylacetate and methanol will afford the expected amine 2,3,5-tri-O-benzyl-1,4-dideoxy-1,4-{[2-(1-O-methyl-1-α-D-arabinofuranosyl)ethyl]-imino}-L-arabinitol.

Example 43

Preparation of

1,4-DIDEOXY-1,4-{[2-(1-O-METHYL-1-α-D-ARABINOFURANOSYL)ETHYL]-IMINO}-L-ARABINITOL

The amine 2,3,5-tri-O-benzyl-1,4-dideoxy-1,4-{[2-(1-O-methyl-1-α-D-arabinofuranosyl)ethyl]imino}-L-arabinitol (0.4 g, 0.69 mmol) is dissolved in acetic acid (20 mL). Palladium 10% on charcoal (0.2 g) is added and the mixture is hydrogenated during 4 days at 3 atmosphere. The catalyst is removed by filtration and the solvents are evaporated under reduced pressure. The residue is dissolved in water and passed through

a column of Amberlyst A26 $OH^{\ominus}$ form. Water is evaporated under reduced pressure. Flash chromatography on silica gel and elution with a graded mixture of chloroform, methanol and water will afford the expected amine 1,4-dideoxy-1,4-{[2-(1-O-methyl-1-α-D-arabinofuranosyl)ethyl]-imino}-L-arabinitol.

## Example 44

Preparation of

METHYL 6-O-(4-O-ACETYL-2,3,6-TRI-O-BENZYL-α-D-GALACTOPYRANOSYL)-2,3,4-TRI-O-BENZYL-α-D-GLUCOPYRANOSIDE

Ethereal silver perchlorate (0.08 M, 76.9 mL, 6.15 mmol) is added with stirring at -30° C to a solution of methyl 2,3,4- tri-O-benzyl-α-D-glucopyranoside (2.078 g, 4.48 mmol), 4-O-acetyl-2,3,6-tri-O-benzyl-α-D-galactopyranosyl chloride (2.859 g, 5.6 mmol) and 2,4,6-trimethylpyridine (0.81 mL, 6.15 mmol) in ether (20 mL). The mixture is stirred 15 min at -30° C and silver chloride is precipitated. The mixture is filtered through a celite pad, the solids are washed with ether, the filtrate is concentrated under reduced pressure. The residue is dissolved in methylene chloride and the organic layer is washed successively with aqueous sodium thiosulfate and water. The organic layer is dried over sodium sulfate, filtered and concentrated under reduced pressure to afford a foam. Flash chromatography on silica gel and elution with a graded mixture of hexane and ethyl acetate will afford methyl 6-O-(4-O-acetyl-2,3, 6-tri-O-benzyl-α-D-galactopyranosyl)-2,3,4-tri-O-benzyl-α-D-glucopyranoside.

## Example 45

Preparation of

METHYL 2,3,4-TRI-O-BENZYL-6-O-(2,3,6-TRI-O-BENZYL-α-D-GALACTOPYRANOSYL)-α-D-GLUCOPYRANOSIDE

Methyl 6-O-(4-O-acetyl-2,3,6-tri-O-benzyl-α-D-galactopyranosyl)-2,3,4-tri-O-benzyl-α-D-glucopyranoside (2.314 g, 2.45 mmol) is dissolved in hot toluene (20 mL) and methanol (80 mL) is added, followed by a few drops of 1 M. methanolic sodium methoxide. The mixture is stirred at room temperature during 2 h. The reaction mixture is made neutral with Amberlite IR 120 ($H^+$) resin, filtered and concentrated under reduced pressure and will afford methyl 2,3,4-tri-O-benzyl-6-O(2,3,6-tri-O-benzyl-α-D-galactopyranosyl)-α-D-glucopyranoside (2.21 g, 100%).

## Example 46

Preparation of

METHYL 6-O-(2,3,6-TRI-O-BENZYL-4-O-TRIFLUOROMETHYLSULFONYL-α-D-GALACTOPYRANOSYL)-2,3,4-TRI-O-BENZYL-α-D-GLUCOPYRANOSIDE

To a solution of dry pyridine (0.45 mL) in dry methylene chloride (40 mL) cooled to -15° C is added trifluoromethanesulfonic anhydride (0.83 mL). The mixture is stirred during 15 min at -10° C, then methyl 2,3,4-tri-O-benzyl-6-O-(2,3,6-tri-O-benzyl-α-D-galactopyranosyl)-α-D-glucopyranoside (2.21 g, 2.46 mmol) in methylene chloride (10 mL) is added. The mixture is washed with water. The organic layer is dried over sodium sulfate, filtered and concentrated under reduced pressure to afford an oil which will be the expected triflate methyl 6-0-(2,3,6-tri-O-benzyl-4-O-trifluoromethylsulfonyl-α-D-galactopyranosyl)-2,3 ,4-tri-O-benzyl-α-D-glucopyranoside.

## Example 47

Preparation of

2,3,5-TRI-O-BENZYL-1,4-DIDEOXY-N-[2,3,6-TRI-O-BENZYL-DEOXY-1-(2,3,4-TRI-O-BENZYL-1-O-METHYL-6-O-α-D-GLUCOPYRANOSYL)-α-D-GLUCOPYRANOSYL]-1,4-IMINO-L-ARABINITOL

A solution of methyl 6-O-(2,3,6-tri-O-benzyl-4-O-tri-fluoromethysulfonyl-α-D-galactopyranosyl)-2,3,4-tri-O-benzyl-α-D-glucopyranoside (1.6 g, 1.55 mmol) and 2,3,5-tri-O-benzyl-1,4-dideoxy-1,4-imino-L-arabinitol (0.625 g, 1.55 mmol) in ethanol-free chloroform (50 mL) is refluxed under nitrogen during 48 h. The mixture is diluted in methylene chloride and washed successively with a saturated aqueous solution of sodium bicarbonate and saturated brine. The organic layer is dried over sodium sulfate, filtered and concentrated under reduced pressure to afford a foam. Flash chromatography on silica gel and elution with a graded mixture of hexane and ethyl acetate will afford the expected amine 2,3,5-tri-O-benzyl-1,4-dideoxy-N-[2,3,6-tri-O-benzyl-4-deoxy-1-(2,3,4-tri-O-benzyl-1-O-methyl-6-O-a-D-gluco-pyranosyl)-α-D-glucopyranosyl] -1,4-imino-L-arabinitol.

## Example 48

Preparation of

1,4-DIDEOXY-N-[4-DEOXY-1-(1-O-METHYL-6-O-α-D-GLUCOPYRANOSYL)-α-D-GLUCOPYRANOSYL]-1,4-IMINO-L-ARABINITOL

2,3,5-tri-O-benzyl-1 ,4-dideoxy-N-[2,3,6-tri-O-benzyl-4-deoxy-1-(2,3,4-tri-O-benzyl-1-O-methyl-6-O-α-D-gLucopyranosyl)-α-D-glucopyranosyl]-1,4-imino-L-arabinitol (1.2 g, 0.936 mmol) is dissolved in acetic acid (30 mL). Palladium 10% on charcoal (0.6 g) is added. The mixture is hydrogenated druing 4 days at 3 atmospheres. The catalyst is removed by filtration and the solvents are evaporated under reduced pressure. The residue is dissolved in water and passed through a column of Amberlyst A26 $OH^{\ominus}$ form. Water is evaporated under reduced pressure and flash chromatography on silica gel and elution with a graded mixture of chloroform, methanol and water will afford the expected amine 1,4-dideoxy-N-[4-deoxy-1-(1-O-methyl-6-O-α-D-glucopyranosyl)-α-D-glucopyranosyl]-1,4-imino-L-arabinitol (0.22 g, 49%) as an amorphous solid.

## Example 49

Preparation of

2,3,6-TRI-O-BENZYL-4-DEOXY-4-HYDROXYMETHYL-D-GLUCOPYRANOSE

Methyl 2,3,6-tri-O-benzyl-4-deoxy-4-hydroxymethyl-α-D-glucopyranoside (4.78 g, 10 mmol) is dissolved at 0° C in a 9:1 mixture of trifluoracetic acid and water (50 mL). The mixture is stirred overnight at 0° C. The solvents are evaporated under reduced pressure without heating. The residue is dissolved in ethyl acetate and washed successively with sodium bicarbonate and brine. The organic layer is dried over sodium sulfate, filtered and concentrated under reduced pressure to afford an oil. Flash chromatography on silica gel and elution with a graded mixture of ethyl acetate and hexane will afford 2,3,6-tri-O-benzyl-4-deoxy-4-hydroxy-methyl-D-glucopyranose.

Example 50

Preparation of

ACETYL 2,3,6-TRI-O-BENZYL-4-DEOXY-4-ACETYLOXYMETHYL-D-GLUCOPYRANOSIDE

2,3,6-tri-O-benzyl-4-deoxy-4-hydroxymethyl-D-glucopyranose (5.10 g, 9.30 mmol) is dissolved in dry pyridine (25 mL) and acetic anhydride (5 mL) is added. The mixture is stirred during 24 h at room temperature. The solvent is evaporated under high vacuum. The residue is dissolved in ethyl acetate and washed with water. The organic Layer is dried over sodium sulfate, filtered and concentrated under reduced pressure and will afford the expected diacetate acetyl 2,3,6-tri-O-benzyl-4-deoxy-4-acetyloxymethyl-D-gluco-pyranoside as an oil which is used without purification.

Example 51

Preparation of

2,3,6-TRI-O-BENZYL-1,4-DIDEOXY-4-ACETYLOXYMETHYL-D-GLUCOPYRANOSYL CHLORIDE

Acetyl 2,3,6-tri-O-benzyl-4-deoxy-4-acetyloxymethyl-D-glucopyranoside (5.10 g, 9.30 mmol) in ether (10 mL) is treated with ethereal hydrogen chloride (0.2 g|mL, 25 mL). The mixture is stirred at room temperature during 48 h. The solvents are evaporated under reduced pressure to afford an oil. Flash chromatography on silica gel and elution with a graded mixture of carbon tetrachloride and ethyl acetate will afford 2,3,6-tri-O-benzyl-1,4-dideoxy-4-acetyloxymethyl-D-glucopyranosyl chloride.

Example 52

Preparation of

METHYL 4-O-(2,3,6-TRI-O-BENZYL-4-DEOXY-4-ACETYLOXYMETHYL-α-D-GLUCOPYRANOSYL)-2,3,6-TRI-O-BENZYL-α-D-GLUCOPYRANOSIDE

Ethereal silver perchlorate (0.08 M, 9.58 mL, 7.67 mmol) is added with stirring at -30° C to a solution of methyl 2,3,6-tri-O-benzyl-α-D-glucopyranoside (2.592 g, 5.59 mmol), 2,3,6-tri-O-benzyl-1,4-dideoxy-4-acetyloxymethyl-D-gluco-pyranosyl chloride (3.661 g, 6.98 mmol) in ether (20 mL). The mixture is stirred 15 min at -30° C and silver chloride precipitated. The mixture is filtered through a celite pad, the solids are washed with ether, the filtrate is concentrated under reduced pressure. The residue is dissolved in methylene chloride and the organic layer is washed successively with aqueous sodium thiosulfate and water. The organic Layer is dried over sodium sulfate, filtered and concentrated under reduced pressure. Flash chromatography on silica gel and elution with a graded mixture of hexane and ethyl acetate will afford methyl 4-O-(2,3,6-tri-O-benzyl-4-deoxy-4-acetyloxymethyl-α-D-glucopyranosyl)-2,3,6-tri-O-benzyl-α-D-glucopyranoside.

## Example 53

Preparation of

METHYL 4-O-(2,3,6-TRI-O-BENZYL-4-DEOXY-4-HYDROXYMETHYL-α-D-GLUCOPYRANOSYL)-2,3,6-TRI-O-BENZYL-α-D-GLUCOPYRANOSIDE

Methyl 4-O-(2,3,6-tri-O-benzyl-4-deoxy-4-acetyloxy-methy-α-D-glucopyranosyl)-2,3,6-tri-O-benzyl-α-D-glucopyranoside (3.19 g, 3.35 mmol) is dissolved in hot toluene (20 mL) and methanol (80 mL) is added, followed by a few drops of 1 M. methanolic sodium methoxide. The mixture is stirred at room temperature during 2 h. The reaction mixture is made neutral with Amberlite IR 120 (H+) resin, filtered and concentrated under reduced pressure and will afford methyl 4-O-(2,3,6-tri-O-benzyl-4-deoxy-4-hydroxymethyl-α-D-glucopyranosyl)-2,3,6-tri-O-benzyl-α-D-glucopyranoside.

## Example 54

Preparation of

METHYL 4-O-(2,3,6-TRI-O-BENZYL-4-DEOXY-4-TRIFLUOROMETHYLSULFONYLOXMETHYL-α-D-GLUCOPYRANOSYL)-2,3,6-TRI-O-BENZYL-α-D-GLUCOPYRANOSIDE

To a solution of dry pyridine (0.6 mL) in dry methylene chloride (50 mL) cooled to -15° C is added trifluoromethanesulfonic anhydride (1.12 mL). The mixture is stirred during min at -10° C, then methyl 4-O-(2,3,6-tri-O-benzyl-4-deoxy-4-hydroxymethyl-α-D-glucopyranosyl)-2,3,6-tri-O-benzyl-α-D-glucopyranoside (3.049g, 3.35 mmol) in methylene chloride (15 mL) is added. The mixture is washed with water. The organic layer is dried over sodium sulfate, filtered and concentrated under reduced pressure and will afford an oil (3.42 g, 98%) which will be the expected triflate methyl 4-O-(2,3,6-tri-O-benzyl-4- deoxy-4-trifluoromethylsulfonyloxymethyl-α-D-glucopyranosyl)-2,3,6-tri-O-benzyl-α-D-glucopyranoside.

## Example 55

Preparation of

2,3,5-TRI-O-BENZYL-1,4-DIDEOXY-N-{[2,3,6-TRI-O-BENZYL-4-DEOXY-1-(2,3,6-TRI-O-BENZYL-4-O-α-D-GLUCOPYRANOSYL)-4-α-D-GLUCOPYRANOSYL]METHYL}1,4-IMINO-L-ARABINITOL

A solution of methyl 4-O-(2,3,6-tri-O-benzyl-4-deoxy-4-trifluoromethylsulfonyloxymethyl-α-D-glucopyranosyl)-2,3,6-tri-O-benzyl-α-D-glucopyranoside (1.82 g, 1.75 mmol) and 2,3,5-tri-O-benzyl-1,4-dideoxy-1,4-imino-L-arabinitol (0.705 g, 1.75 mmol) in ethanol-free chloroform (50 mL) is refluxed under nitrogen during 48 h. The mixture is diluted in methylene chloride and washed successively with a saturated brine. The organic layer is dried over sodium sulfate, filtered and concentrated under reduced pressure and will afford a foam. Flash chromatography on silica gel and elution with a graded mixture of hexane and ethyl acetate will afford the expected amine 2,3,5-tri-O-benzyl-1,4-dideoxy-N-{[2,3,6-tri-O-benzyl-4-deoxy-1-(2,3,6-tri-O-benzyl-1-O-methyl-4-O-α-D-glucopyranosyl)-4-α-D-glucopyranosyl]methyl}1,4-imino-L-arabinitol.

## Example 56

Preparation of

1,4-DIDEOXY-N-{[4-DEOXY-1-(1-O-METHYL-4-O-α-D-GLUCOPYRANOSYL)-4-α-D-GLUCOPYRANOSYL]-METHYL}1,4-IMINO-L-ARABINITOL

2,3,5-tri-O-benzyl-1,4-dideoxy-N-{[2,3,6-tri-O-benzyl-4-deoxy-1-(2,3,6-tri-O-benzyl-1-O-methyl-4-O-α-D-glucopyranosyl)-4-α-D-glucopyranosyl]methyl}1,4-imino-L-arabinitol (1.3 g, 1.146 mmol) is dissolved in acetic acid (40 mL). Palladium 10% on charcoal (0.6 g) is added. The mixture is hydrogenated during 4 days at 3 atmosphere. The catalyst is removed by filtration and the solvents are evaporated under reduced pressure. The residue is dissolved in water and passed through a column of Amberlyst A26 $OH^{\ominus}$. Water is evaporated under reduced pressure and flash chromatography on silica gel and elution with a graded mixture of chloroform, methanol and water will afford the expected amine 1,4-dideoxy-N-{[4-deoxy-1-(1-O-methyl-4-O-α-D-glucopyranosyl)-4-α-D-glucopyranosyl]methyl}1,4-imino-L-arabinitol.

## Example 57

Preparation of

METHYL 6-O-(2,3,6-TRI-O-BENZYL-4-DEOXY-4-ACETYLOXYMETHYL-α-D-GLUCOPYRANOSYL)-2,3,4-TRI-O-BENZYL-α-D-GLUCOPYRANOSIDE

Ethereal silver perchlorate (0.08 M, 76.7 mL, 6.13 mmol) is added with stirring at -30° C to a solution of methyl 2,3,4-tri-O-benzyl-α-D-glucopyranoside (2.074 g, 4.472 mmol), 2,3,6-tri-O-benzyl-1,4-dideoxy-4-acetyloxymethyl-D-gluco-pyranosyl chloride (6.13 mmol) and 2,4,6-trimethylpyridine (0.80 mL, 6.13 mmol) in ether (20 mL). The mixture is stirred 15 min at -30° C and silver chloride precipitated. The mixture is filtered through a celite pad, the solids are washed with ether, the filtrate is concentrated under reduced pressure. The residue is dissolved in methylene chloride and the organic layer is washed successively with aqueous sodium thiosulfate and water. The organic layer is dried over sodium sulfate, filtered and concentrated under reduced pressure to afford a foam. Flash chromatography on silica gel and elution with a graded mixture of hexane and ethyl acetate will afford methyl 6-O-(2,3,6-tri-O-benzyl-4-deoxy-4-acetyloxymethyl-α-D-glucopyranosyl)-2,3,4-tri-O-benzyl-α-D-glucopyranoside.

Example 58

Preparation of

METHYL 6-O-(2,3,6-TRI-O-BENZYL-4-DEOXY-4-HYDROXYMETHYL-α-GLUCOPYRANOSYL)-2,3,4-TRI-O-BENZYL-α-D-GLUCOPYRANOSIDE

Methyl 6-O-(2,3,6-tri-O-benzyl-4-deoxy-4-acetyloxy-methyl-α-D-glucopyranosyl)-2,3,4-tri-O-benzyl-α-D-glucopyranoside (2.469 g, 2.593 mmol) is dissolved in hot toluene (20 mL) and methanol (80 mL) is added, followed by a few drops of 1 M. methanolic sodium methoxide. The mixture is stirred at room temperature during 2 h. The reaction mixture is made neutral with Amberlite IR 120 ($H^+$) resin, filtered and concentrated under reduced pressure and will afford methyl 6-O-(2,3,6-tri-O-benzyl-4-deoxy-4-hydroxymethyl-α-D-glucopyranosyl)-2,3,4-tri-O-benzyl-α-D-glucopyranoside.

Example 59

Preparation of

METHYL 6-O-(2,3,6-TRI-O-BENZYL-4-DEOXY-4-TRIFLUOROMETHYLSULFONYLOXYMETHYL-α-D-GLUCOPYRANOSYL-2,3,4-TRI-O-BENZYL-α-D-GLUCOPYRANOSIDE

To a solution of dry pyridine (0.46 mL) in dry methylene chloride (40 mL) cooled to -15° C is added trifluoromethanesulfonic anhydride (0.86 mL). The mixture is stirred during 15 min at -10° C, then methyl 6-O-(2,3,6-tri-O-benzyl-4-deoxy-4-hydroxymethyl-α-D-glucopyranosyl)2,3,4-tri-O-benzyl-α-D-glucopyranoside (2.36 g, 2.593 mmol) in methylene chloride (10 mL) is added. The mixture is stirred during 1.5 h at -10° C. The reaction mixture is washed with water. The organic layer is dried over sodium sulfate, filtered and concentrated under reduced pressure which will afford an oil, the expected tri flate methyl 6-O-(2,3,6-tri-O-benzyl-4-deoxy-4-trifluoromethylsulfonyloxymethyl-α-D-glucopyranosyl)-2,3,4-tri-O-benzyl-α-D-glucopyranoside.

Example 60

Preparation of

2,3,5-TRI-O-BENZYL-1,4-DIDEOXY-N-{[2,3,6-TRI-O-BENZYL-4-DEOXY-1-(2,3,4-TRI-O-BENZYL-1-O-METHYL-6-O-α-D-GLUCOPYRANOSYL-4-α-D-GLUCOPYRANOSYL]METHYL}1,4-IMINO-L-ARABINITOL

A solution of methyl 6-O-(2,3,6-tri-O-benzyl-4-deoxy-4-trifluoromethylsulfonyloxymethyl-α-D-glucopyranosyl)-2,3,4-tri-O-benzyl-α-D-glucopyranoside (1.8 g, 1.72 mmol) and 2,3,5-triO-benzyl-1,4-dideoxy-1,4-imino-L-arabinitol (0.693 g, 1.72 mmol) in ethanol-free chloroform (50 mL) is refluxed under nitrogen during 48 h. The mixture is diluted in methylene chloride and washed successively with a saturated aqueous solution of sodium bicarbonate and saturated brine. The organic layer is dried over sodium sulfate, filtered and concentrated under reduced pressure to afford a foam. Flash chromatography on silica gel and elution with a graded mixture of hexane and ethyl acetate will afford the expected amine 2,3,5-tri-O-benzyl-1,4-dideoxy-N-{[2,3,6-tri-O-benzyl-4-deoxy-1-(2,3,4-tri-O-benzyl-1-O-methyl-6-O-α-D-glucopyranosyl)-4-α-D-glucopyranosyl]methyl}1,4-imino-L-arabinitol.

Example 61

Preparation of

1,4-DIDEOXY-N-{[4-DEOXY-1-(1-O-METHYL-6-O-α-D-GLUCOPYRANOSYL-4-α-D-GLUCOPYRANOSYL]-METHYL}1,4-IMINO-L-ARABINITOL

2,3,5-tri-O-benzyl-1,4-dideoxy-N-{[2,3,6-tri-O-benzyl-4-deoxy-1-(2,3,4-tri-O-benzyl-1-O-methyl-6-O-α-D-glucopyranosol)- 4-α-D-glucopyranosyl]methyl}1,4-imino-L-arabinitol (1.3 g, 1.003 mmol) is dissolved in acetic acid (30 mL). Palladium 10% on charcoal (0.6 g) is added. The mixture is hydrogenated during 4 days at 3 atmosphere. The catalyst is removed by filtration and the solvents are evaporated under reduced pressure. The residue is dissolved in water and passed through a column of Amberlyst A26 $OH^{\ominus}$. Water is evaporated under reduced pressure and flash chromatography on silica gel and elution with a graded mixture of chloroform, methanol and water will afford the expected amine 1,4-dideoxy-N-{[4-deoxy-1-(1-O-methyl-6-O-α-D-glucopyranosyl)-4-α-D-glucopyranosyl]methyl}1,4-imino-L-arabinitol.

Example 62

Preparation of

1,5-DIDEOXY-1,5-(6-DEOXY-6-D-GLUCOPYRANOSYL) IMINO-L-ARABINITOL

1,4-dideoxy-1,4-(6-deoxy-1-O-methyL-6-α-D-glucopyranosyl)-imino-L-arabinitol (0.150 g, 0.485 mmol) is dissolved in a 1:1 mixture of water and trifluoroacetic acid (10 mL). The mixture is stirred during 24 h at 0° C. The solvents are evaporated under reduced pressure to afford a foam. Chromatography on Amberlyst A26 $OH^{\ominus}$ form will afford the expected amine 1,5-dideoxy-1,5-(6-deoxy-6-D-glucopyranosyl)imino-L-arabinitol.

Enzymes which catalyze the hydrolysis of complex carbohydrates, e.g. α-glycosidases, convert non-absorbable carbohydrates into absorbable sugars. The rapid action of these enzymes, particularly following the intake of high levels of carbohydrates, lead to acute high levels in blood glucose which, in the case diabetics, lead to undesirable manifestations, thus it has been a long-sought goal to find compounds which will obviate the hyperglicemia caused by dietary improprieties. Similarly, in the case of obesity the control of high levels of blood glucose, with its subsequent conversion to fat, caused by the catalysis of carbohydrates has inspired the quest for compounds which will obviate the problems associated with dietary improprieties.

The compounds of this invention (I) are potent and long-lasting inhibitors of α-glucosidase and, by standard laboratory methods for determining serum glucose levels, are shown to be useful for the treatment of disease states caused by the underutilization and/or overproduction of serum glucose without adversely affecting the rate of transport across cell membranes. Thus, the compounds are useful in the treatment of diabetes and obesity.

In the practice of this invention, an effective amount of a compound of this invention is that amount required to reduce the amount of serum glucose (relative to a control) following the ingestion of carbohydrates convertible to absorbable glucose. The specific dosage for the treatment of any specific patient suffering from either disease state will depend upon such factors as size, type and age of the patient as well as the severity of the disease state, all of which are factors normally familiar to and considered by

the attending diagnostician treating the patient. Generally, the compounds are to be administered orally at a dose of 0.2 to 20 milligrams per kilogram of body weight (MPK) with a dose of 0.5 to 5 MPK being preferred. The compounds preferable are to be administered orally at mealtimes in single or multiple unit doses containing 25 mg to 250 mg. Of course, in the treatment of obesity, the term includes the practice of the disease as well as continuel administration of dose regimens suitable for the maintenance of the desired weight for the patient.

It is also to be found that the compounds of the instant invention (I) will exert an inhibitory effect on glycosidase enzymes that are essential for elaboration of the final structure of the oligosaccharide side-chains of glyco-proteins, particularly the HIV (gp 120) glycoprotein. Suitable assay techniques, e.g. syncytial formation, the reverse transcriptase assay, immunofluorescence tests and election microscopy, may be used to evaluate the effects on HIV viral growth and for determining dose regimens. Antiviral effects may be confirmed by immunofluorescence with serum for virally infected patients. In the treatment of the HIV related disease states, as well as other retroviral glyco-protein-related disease states, unlike the treatment of diabetes and obesity, the compounds of this invention may be administered by parenteral means; specific doses being within the above stated dose range for treatment of diabetes and obesity.

In practising the end-use application of the compounds of this invention, the compounds are preferably incorporated in a pharmaceutical formulation comprising a pharmaceutical carrier in admixture with a compound of this invention. The term "pharmaceutical carrier" refers to known pharmaceutical excipients useful in formulating pharmaceutically active compounds for internal administration to animals, and which are substantially non-toxic and non-sensitizing under conditions of use. The compositions can be prepared by known techniques for the preparation of tablets, capsules, elixirs, syrups, emulsions, dispersions and wettable and effervescent powders, and can contain suitable excipients known to be useful in the preparation of the particular type of composition desired. Suitable pharmaceutical carriers and formulation techniques are found in standard texts, such as Remington's Pharmaceutical Sciences, Mack Publishing Company, Easton, Pa.

## Claims

1. 1,4-Dideoxy-1,4-imino-L-arabinitol derivatives of the formula

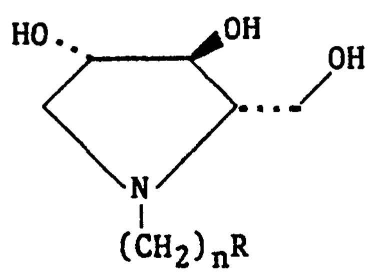

I

and the pharmaceutically acceptable acid addition salts thereof wherein n is 1 or 2 and R is a glycosyl moiety containing from 1 to 3 hexose or pentose units.

2. A compound of Claim 1 wherein R is a glucosyl, galactosyl, fucosyl, fructosyl, mannosyl, ribosyl, arabinosyl, xylosyl, allosyl, altrosyl, gulosyl, indosyl, talosyl,lyxosyl, isomaltosyl, trehalosyl $\alpha$ and $\beta$ cellobiosyl, maltosyl, maltotriosyl or cellotriosyl radical.

3. A compound of Claim 1 wherein R is 6-glucosyl, 4-glucosyl, 1-fructosyl, 6-fructosyl, 6-maltosyl, 4-maltosyl, 6-isomaltosyl or 4-isomaltosyl.

4. A compound of Claim 1, said compound being 1,4-dideoxy-1,4-[(6-deoxy-1-O-methyl-6-$\alpha$-D-glucopyranosyl)imino]-L-arabinitol.

5. A compound of Claim 1, said compound being 1,4-dideoxy-1,4-[(6,7-dideoxy-1-O-methyl-7-$\alpha$-D-glucoheptopyranosyl)imino]-L-arabinitol.

6. A compound of Claim 1, said compound being 1,4-dideoxy-1,4-[(1-deoxy-2-O-methyl-$\alpha$-D-fructofuranosyl)imino]-L-arabinitol.

7. A compound of Claim 1, said compound being 1,4-dideoxy-1,4-[(4-deoxy-1-O-methyl-4-$\alpha$-D-glucopyranosyl)imino]-L-arabinitol.

8. A compound of Claim 1, said compound being 1,4-dideoxy-N-[6-deoxy-1-(1-O-methyl-6-O-α-D-glucopyranosyl)-α-D-glucopyranosyl]-1,4-imino-L-arabinitol.

9. A compound of Claim 1, said compound being 1,4-dideoxy-N-[6,7-dideoxy-1(1-O-methyl-6-O-α-D-glucopyranosyl)-α-D-glucoheptopyranosyl]-1,4-imino-L-arabinitol.

10. A compound of Claim 1, said compound being 1,4-dideoxy-1,4-[(4-deoxy-1-O-methyl-4-α-D-glucopyranosyl)methylimino]-L-arabinitol.

11. A compound of Claim 1, said compound being 1,4-dideoxy-N-[4-deoxy-1-(1-O-methyl-4-O-α-D-glucopyranosyl)-α-D-glucopyranosyl]-1,4-imino-L-arabinitol.

12. A compound of Claim 1, said compound being 1,4-dideoxy-1,4-{[2-(1-O-methyl-1-α-D-arabinofuranosyl)ethyl]imino}-L-arabinitol.

13. A compound of Claim 1, said compound being 1,4-dideoxy-N-[4-deoxy-1-(1-O-methyl-6-O-α-D-glucopyranosyl)-α-D-glucopyranosyl]-1,4-imino-L-arabinitol.

14. A compound of Claim 1, said compound being 1,4-dideoxy-N-[4-deoxy-1-(1-O-methyl-4-O-α-D-glucopyranosyl)-4-α-D-glucopyranosyl]methyl}1,4-imino-L-arabinitol.

15. A compound of Claim 1, said compound being 1,4-dideoxy-N-{[4-deoxy-1-(1-O-methyl-6-O-α-D-glucopyranosyl)-4-α-D-glucopyranosyl]methyl}1,4-imino-L-arabinitol.

16. A compound of Claim 1, said compound being 1,4-dideoxy-1,4-(6-deoxy-6-D-glucopyranosyl)imino-L-arabinitol.

17. The derivatives according to anyone of claims 1 to 16 for use as pharmaceutically active compound.

18. Use of the derivaties according to anyone of claims 1 to 16 for the preparation of drugs useful for inhibiting α-glucosidase enzymes.

19. Use of the derivatives according to anyone of claims 1 to 16 for the preparation of drugs useful for treating diabetes.

20. Use of the derivatives according to anyone of claims 1 to 16 for the preparation of drugs useful for controlling obesity by reducing the amount of systematically absorbable glucose following ingestion of food substances capable of being enzymatically converted to glucose.

21. The process for preparing compounds of the formula

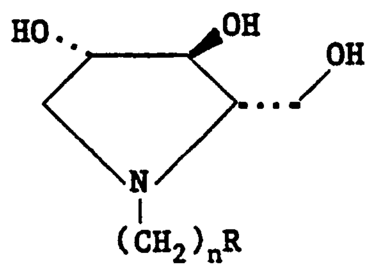

I

and the pharmaceutically acceptable salts thereof, wherein
n is 1 or 2, and
R is a glycosyl moiety containing from 1 to 3 hexose or pentose units, which comprises reactions (a) and (b), said reaction (a) comprising the condensation of a compound of the formula

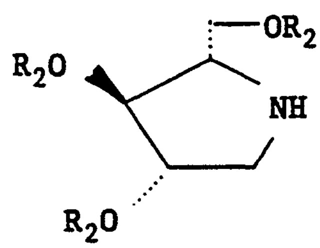

(II)

with a compound of formula $X-(CH_2)_nR_1$, said reaction (b) comprising the condensation of a compound of Formula V

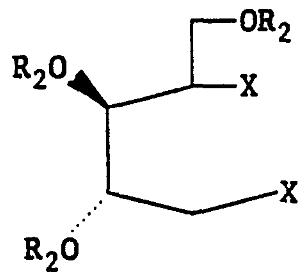

(V)

with a compound of Formula $H_2N-(CH_2)_nR_1$, followed by the removal of any reaction protecting groups of the condensation products of reaction (a) and (b),

and optionnaly converting said de-protected condensation products to their pharmaceutically acceptable salts, wherein

$R_1$ is a glycosyl moiety containing from 1 to 3 hexose or pentose units, the hydroxy radicals of which may bear a reaction-protecting group,

X is chloro, bromo, iodo or an activated oxygen radical,

n is one or two,

$R_2$ is H or a reaction protecting group, with the proviso that when X is an activated oxygen moiety, $R_2$ is other than H and the free hydroxy radicals of the glycosyl moieties must bear a reaction-protection group.

European Patent Office

# EUROPEAN SEARCH REPORT

Application number

EP 90400835.6

## DOCUMENTS CONSIDERED TO BE RELEVANT

| Category | Citation of document with indication, where appropriate, of relevant passages | Relevant to claim | CLASSIFICATION OF THE APPLICATION (Int Cl 5) |
|---|---|---|---|
| A | EP - A2 - 0 210 639 (WARMER LAMBERT COMP.) * Claims * | 1-17, 21 | C 07 H 15/26<br>A 61 K 31/70 |
| A | EP - A2 - 0 155 164 (SUMITOMO PHARM. COMP.) * Claims * | 1-17, 21 | |
| P,A | EP - A2 - 0 345 104 (MERZELL DOW PHARM. COMP.) * Reaction scheme C; claims * | 1-21 | |
| D,A | TETRAHEDRON LETTERS, vol. 26, no. 19, 1985, Fleet et al. "Potent competitive inhibition of alpha-galactosidase and alpha-glucosidase activity by 1,4-dideoxy-1,4-iminopentitols: Syntheses of 1,4-dideoxy-1,4-imino-d-lyxitol and of both enantiomers of 1,4-dideoxy-1,4-iminoarabinitol", pages 3127-3130 * Abstract; pages 3129/3130 (compounds 15-18) * | 1,18, 21 | TECHNICAL FIELDS SEARCHED (Int Cl 5)<br>C 07 H 15/00<br>C 07 H 19/00 |
| D,A | TETRAHEDRON, vol. 44, no. 9, 1988, Fleet et al. "Polyhydroxylated pyrrolidines from sugar lactones: Synthesis of 1,4-dideoxy-1,4-imino-d-glucitol from d-galactonolactone and syntheses of 1,4-dideoxy-1,4-imino-d-allitol, 1,4-dideoxy-1,4-imino-d-ribitol, and (2S,3R,4S)-3,4-dihydroxyproline from d-gul- | 1,18, 21 | |

The present search report has been drawn up for all claims

| Place of search | Date of completion of the search | Examiner |
|---|---|---|
| VIENNA | 19-06-1990 | JANISCH |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application number

-2-
EP 90400835.6

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int Cl.5) |
| | onolactone",<br>pages 2637-2647<br>* Pages 2637,2638 *<br>---- | | |
| | | | TECHNICAL FIELDS SEARCHED (Int Cl.5) |

The present search report has been drawn up for all claims

| Place of search | Date of completion of the search | Examiner |
|---|---|---|
| VIENNA | 19-06-1990 | JANISCH |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document